(19) Europäisches Patentamt
European Patent Office
Office européen des brevets

(11) EP 0 727 211 A1

(12) EUROPEAN PATENT APPLICATION

(43) Date of publication:
21.08.1996 Bulletin 1996/34

(21) Application number: 96200270.5

(22) Date of filing: 07.02.1996

(51) Int Cl.6: A61K 31/50, A61K 31/12, A61K 31/435, A61K 31/195, A61K 31/165, A61K 31/40

(84) Designated Contracting States:
AT BE CH DE DK ES FR GB GR IE IT LI LU MC NL PT SE

(30) Priority: 10.02.1995 US 386381
07.03.1995 US 400220
30.06.1995 US 497357
11.10.1995 US 541080

(71) Applicant: SMITHKLINE BEECHAM CORPORATION
Philadelphia Pennsylvania 19103 (US)

(72) Inventor: Dunnington, Damien John
Lafayette Hill, PA 19444 (US)

(74) Representative: Giddings, Peter John, Dr. et al
SmithKline Beecham plc
Corporate Intellectual Property
SB House
Great West Road
Brentford, Middlesex TW8 9BD (GB)

# (54) Use of src SH2 specific compounds to treat a bone resorption disease

(57) Invented is a method of treating a bone resorption disease in a subject which comprises administering to the subject a therapeutically effective amount of a compound which binds to a human src SH2 domain with a binding affinity greater than fifty-fold higher than the binding affinity with which the compound binds to a human lck SH2 domain and a human fyn SH2 domain, and, binds to a human hcp SH2 domain, a human Grb2 SH2 domain, a human SH-PTP2 SH2 domain and a human p85 SH2 domain with a binding affinity which is greater than fifty-fold lower than the binding affinity with which the compound binds to such src SH2 domain.

EP 0 727 211 A1

SEQUENCE LISTING

(1) GENERAL INFORMATION

(i) APPLICANT: DUNNINGTON, DAMIEN

(ii) TITLE OF THE INVENTION: USE OF SRC SH2 SPECIFIC COMPOUNDS TO TREAT A BONE RESORPTION DISEASE

(iii) NUMBER OF SEQUENCES: 26

(iv) CORRESPONDENCE ADDRESS:
(A) ADDRESSEE: SmithKline Beecham Corporation
(B) STREET: 709 Swedeland Road
(C) CITY: King of Prussia
(D) STATE: PA
(E) COUNTRY: USA
(F) ZIP: 19406

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ Version 1.5

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: 08/386,381
(B) FILING DATE: 10-FEB-1995

(A) APPLICATION NUMBER: 08/400,220
(B) FILING DATE: 07-MAR-1995

(A) APPLICATION NUMBER: 08/497,357

(B) FILING DATE: 30-JUN-1995

(viii) ATTORNEY/AGENT INFORMATION:

(A) NAME: Dustman, Wayne J

(B) REGISTRATION NUMBER: 33,870

(C) REFERENCE/DOCKET NUMBER: P50323-2S2

(ix) TELECOMMUNICATION INFORMATION:

(A) TELEPHONE: 610-270-5023

(B) TELEFAX:

(C) TELEX:

(2) INFORMATION FOR SEQ ID NO:1:

(i) SEQUENCE CHARACTERISTICS:

(A) LENGTH: 11 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(ix) FEATURE:

(xi) SEQUENCE DESCRIPTION: SEQ ID NO:1:

```
Lys Ser Ile Arg Ile Gln Arg Gly Pro Gly Arg
 1               5                  10
```

(2) INFORMATION FOR SEQ ID NO:2:

(i) SEQUENCE CHARACTERISTICS:

(A) LENGTH: 6 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:2:

His His His His His His
1 5

(2) INFORMATION FOR SEQ ID NO:3:

(i) SEQUENCE CHARACTERISTICS:

(A) LENGTH amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:3:

Gly Ile Leu
1

(2) INFORMATION FOR SEQ ID NO:4:

(i) SEQUENCE CHARACTERISTICS:

(A) LENGTH: 5 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:4:

```
Asp Asp Asp Asp Lys
 1               5
```

(2) INFORMATION FOR SEQ ID NO:5:

(i) SEQUENCE CHARACTERISTICS:

(A) LENGTH: 130 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:5:

```
Met Lys Ser Ile Arg Ile Gln Arg Gly Pro Gly Arg His His His His
 1               5                  10                  15
His His Gly Ile Leu Asp Asp Asp Asp Lys Ala Glu Glu Trp Tyr Phe
```

```
                20                  25                  30
Gly Lys Ile Thr Arg Arg Glu Ser Glu Arg Leu Leu Leu Asn Ala Glu
            35                  40                  45
Asn Pro Arg Gly Thr Phe Leu Val Arg Glu Ser Glu Thr Thr Lys Gly
        50                  55                  60
Ala Tyr Cys Leu Ser Val Ser Asp Phe Asp Asn Ala Lys Gly Leu Asn
65                  70                  75                  80
Val Lys His Tyr Lys Ile Arg Lys Leu Asp Ser Gly Gly Phe Tyr Ile
                    85                  90                  95
Thr Ser Arg Thr Gln Phe Asn Ser Leu Gln Gln Leu Val Ala Tyr Tyr
                100                 105                 110
Ser Lys His Ala Asp Gly Leu Cys His Arg Leu Thr Thr Val Cys Pro
            115                 120                 125
Thr Ser
    130
```

(2) INFORMATION FOR SEQ ID NO:6:

(i) SEQUENCE CHARACTERISTICS:
(A) LENGTH: 134 amino acids
(B) TYPE: amino acid
(C) STRANDEDNESS: single
(D) TOPOLOGY: linear

(ii) MOLECULE TYPE: peptide
(iii) HYPOTHETICAL: NO
(iv) ANTISENSE: NO
(v) FRAGMENT TYPE: internal
(vi) ORIGINAL SOURCE:

(xi) SEQUENCE DESCRIPTION: SEQ ID NO:6:

```
Met Lys Ser Ile Arg Ile Gln Arg Gly Pro Gly Arg His His His His
 1               5                  10                  15
His His Gly Ile Leu Asp Asp Asp Asp Lys Glu Pro Glu Pro Trp Phe
                20                  25                  30
Phe Lys Asn Leu Ser Arg Lys Asp Ala Glu Arg Gln Leu Leu Ala Pro
```

35 40 45

Gly Asn Thr His Gly Ser Phe Leu Ile Arg Glu Ser Glu Ser Thr Ala

50 55 60

Gly Ser Phe Ser Leu Ser Val Arg Asp Phe Asp Gln Asn Gln Gly Glu

65 70 75 80

Val Val Lys His Tyr Lys Ile Arg Asn Leu Asp Asn Gly Gly Phe Tyr

85 90 95

Ile Ser Pro Arg Ile Thr Phe Pro Gly Leu His Glu Leu Val Arg His

100 105 110

Tyr Thr Asn Ala Ser Asp Gly Leu Cys Thr Arg Leu Ser Arg Pro Cys

115 120 125

Gln Thr Gln Lys Pro Gln

130

(2) INFORMATION FOR SEQ ID NO:7:

(i) SEQUENCE CHARACTERISTICS:

(A) LENGTH: 133 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:7:

Met Lys Ser Ile Arg Ile Gln Arg Gly Pro Gly Arg His His His His

1 5 10 15

His His Gly Ile Leu Asp Asp Asp Asp Lys Ser Arg Gly Trp Phe His

20 25 30

Arg Asp Leu Ser Gly Leu Asp Ala Glu Thr Leu Leu Lys Gly Arg Gly

35 40 45

Val His Gly Ser Phe Leu Ala Arg Pro Ser Arg Lys Asn Gln Gly Asp

```
    50                  55                  60
Phe Ser Leu Ser Val Arg Val Gly Asp Gln Val Thr His Ile Arg Ile
65                  70                  75                  80
Gln Asn Ser Gly Asp Phe Tyr Asp Leu Tyr Gly Gly Glu Lys Phe Ala
                85                  90                  95
Thr Leu Thr Glu Leu Val Glu Tyr Tyr Thr Gln Gln Gln Gly Val Leu
            100                 105                 110
Gln Asp Arg Asp Gly Thr Ile Ile His Leu Lys Tyr Pro Leu Asn Cys
        115                 120                 125
Ser Asp Pro Thr Ser
    130
```

(2) INFORMATION FOR SEQ ID NO:8:

(i) SEQUENCE CHARACTERISTICS:
  (A) LENGTH: 224 amino acids
  (B) TYPE: amino acid
  (C) STRANDEDNESS: single
  (D) TOPOLOGY: linear

(ii) MOLECULE TYPE: peptide
(iii) HYPOTHETICAL: NO
(iv) ANTISENSE: NO
(v) FRAGMENT TYPE: internal
(vi) ORIGINAL SOURCE:

(xi) SEQUENCE DESCRIPTION: SEQ ID NO:8:

```
Met Ser Pro Ile Leu Gly Tyr Trp Lys Ile Lys Gly Leu Val Gln Pro
 1               5                  10                  15
Thr Arg Leu Leu Leu Glu Tyr Leu Glu Glu Lys Tyr Glu Glu His Leu
            20                  25                  30
Tyr Glu Arg Asp Glu Gly Asp Lys Trp Arg Asn Lys Lys Phe Glu Leu
        35                  40                  45
Gly Leu Glu Phe Pro Asn Leu Pro Tyr Tyr Ile Asp Gly Asp Val Lys
    50                  55                  60
Leu Thr Gln Ser Met Ala Ile Ile Arg Tyr Ile Ala Asp Lys His Asn
```

```
65                  70                  75                  80
Met Leu Gly Gly Cys Pro Lys Glu Arg Ala Glu Ile Ser Met Leu Glu
                85                  90                  95
Gly Ala Val Leu Asp Ile Arg Tyr Gly Val Ser Arg Ile Ala Tyr Ser
            100                 105                 110
Lys Asp Phe Glu Thr Leu Lys Val Asp Phe Leu Ser Lys Leu Pro Glu
        115                 120                 125
Met Leu Lys Met Phe Glu Asp Arg Leu Cys His Lys Thr Tyr Leu Asn
    130                 135                 140
Gly Asp His Val Thr His Pro Asp Phe Met Leu Tyr Asp Ala Leu Asp
145                 150                 155                 160
Val Val Leu Tyr Met Asp Pro Met Cys Leu Asp Ala Phe Pro Lys Leu
                165                 170                 175
Val Cys Phe Lys Lys Arg Ile Glu Ala Ile Pro Gln Ile Asp Lys Tyr
            180                 185                 190
Leu Lys Ser Ser Lys Tyr Ile Ala Trp Pro Leu Gln Gly Trp Gln Ala
        195                 200                 205
Thr Phe Gly Gly Gly Asp His Pro Pro Lys Ser Asp Leu Val Pro Arg
    210                 215                 220
```

(2) INFORMATION FOR SEQ ID NO:9:

(i) SEQUENCE CHARACTERISTICS:

(A) LENGTH: 225 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:9:

```
Met Ser Pro Ile Leu Gly Tyr Trp Lys Ile Lys Gly Leu Val Gln Pro
```

```
1               5                   10                  15
Thr Arg Leu Leu Leu Glu Tyr Leu Glu Glu Lys Tyr Glu Glu His Leu
            20                  25                  30
Tyr Glu Arg Asp Glu Gly Asp Lys Trp Arg Asn Lys Lys Phe Glu Leu
        35                  40                  45
Gly Leu Glu Phe Pro Asn Leu Pro Tyr Tyr Ile Asp Gly Asp Val Lys
    50                  55                  60
Leu Thr Gln Ser Met Ala Ile Ile Arg Tyr Ile Ala Asp Lys His Asn
65                  70                  75                  80
Met Leu Gly Gly Cys Pro Lys Glu Arg Ala Glu Ile Ser Met Leu Glu
                85                  90                  95
Gly Ala Val Leu Asp Ile Arg Tyr Gly Val Ser Arg Ile Ala Tyr Ser
            100                 105                 110
Lys Asp Phe Glu Thr Leu Lys Val Asp Phe Leu Ser Lys Leu Pro Glu
        115                 120                 125
Met Leu Lys Met Phe Glu Asp Arg Leu Cys His Lys Thr Tyr Leu Asn
    130                 135                 140
Gly Asp His Val Thr His Pro Asp Phe Met Leu Tyr Asp Ala Leu Asp
145                 150                 155                 160
Val Val Leu Tyr Met Asp Pro Met Cys Leu Asp Ala Phe Pro Lys Leu
                165                 170                 175
Val Cys Phe Lys Lys Arg Ile Glu Ala Ile Pro Gln Ile Asp Lys Tyr
            180                 185                 190
Leu Lys Ser Ser Lys Tyr Ile Ala Trp Pro Leu Gln Gly Trp Gln Ala
        195                 200                 205
Thr Phe Gly Gly Gly Asp His Pro Pro Lys Ser Asp Leu Ile Glu Gly
    210                 215                 220
Arg
225
```

(2) INFORMATION FOR SEQ ID NO:10:

(i) SEQUENCE CHARACTERISTICS:

(A) LENGTH: 117 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:10:

```
Ser Ile Gln Ala Glu Glu Trp Tyr Phe Gly Lys Leu Gly Arg Lys Asp
 1               5                  10                  15
Ala Glu Arg Gln Leu Leu Ser Phe Gly Asn Pro Arg Gly Thr Phe Leu
            20                  25                  30
Ile Arg Glu Ser Glu Thr Thr Lys Gly Ala Tyr Ser Leu Ser Ile Arg
        35                  40                  45
Asp Trp Asp Asp Met Lys Gly Asp His Val Lys His Tyr Lys Ile Arg
    50                  55                  60
Lys Leu Asp Asn Gly Gly Tyr Tyr Ile Thr Thr Arg Ala Gln Phe Glu
65                  70                  75                  80
Thr Leu Gln Gln Leu Val Gln His Tyr Ser Glu Arg Glu Arg Ala Ala
                85                  90                  95
Gly Leu Cys Cys Arg Leu Val Val Pro Cys His Lys Gly Met Pro Arg
            100                 105                 110
Leu Thr Asn Ser Ser
        115
```

(2) INFORMATION FOR SEQ ID NO:11:

(i) SEQUENCE CHARACTERISTICS:

(A) LENGTH: 123 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:11:

```
Gly Met Asn Asn Asn Met Ser Leu Gln Asn Ala Glu Trp Tyr Trp Gly
 1               5                  10                  15
Asp Ile Ser Arg Glu Glu Val Asn Glu Lys Leu Arg Asp Thr Ala Asp
            20                  25                  30
Gly Thr Phe Leu Val Arg Asp Ala Ser Thr Lys Met His Gly Asp Tyr
        35                  40                  45
Thr Leu Thr Leu Arg Lys Gly Gly Asn Asn Lys Leu Ile Lys Ile Phe
    50                  55                  60
His Arg Asp Gly Lys Tyr Gly Phe Ser Asp Pro Leu Thr Phe Ser Ser
65                  70                  75                  80
Val Val Glu Leu Ile Asn His Tyr Arg Asn Glu Ser Leu Ala Gln Tyr
                85                  90                  95
Asn Pro Lys Leu Asp Val Lys Leu Leu Tyr Pro Val Ser Lys Tyr Gln
            100                 105                 110
Gln Asp Gln Val Val Lys Glu Asp Asn Ser Ser
        115                 120
```

(2) INFORMATION FOR SEQ ID NO:12:

(i) SEQUENCE CHARACTERISTICS:

(A) LENGTH: 112 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:12:

Met Thr Ser Arg Arg Trp Phe His Pro Asn Ile Thr Gly Val Glu Ala
1 5 10 15

Glu Asn Leu Leu Leu Thr Arg Gly Val Asp Gly Ser Phe Leu Ala Arg
20 25 30

Pro Ser Lys Ser Asn Pro Gly Asp Phe Thr Leu Ser Val Arg Arg Asn
35 40 45

Gly Ala Val Thr His Ile Lys Ile Gln Asn Thr Gly Asp Tyr Tyr Asp
50 55 60

Leu Tyr Gly Gly Glu Lys Phe Ala Thr Leu Ala Glu Leu Val Gln Tyr
65 70 75 80

Tyr Met Glu His His Gly Gln Leu Lys Glu Lys Asn Gly Asp Val Ile
85 90 95

Glu Leu Lys Tyr Pro Leu Asn Cys Ala Asp Gln Phe Ile Val Thr Asp
100 105 110

(2) INFORMATION FOR SEQ ID NO:13:

(i) SEQUENCE CHARACTERISTICS:

(A) LENGTH: 19 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(ix) FEATURE:

(A) NAME/KEY: Other

(B) LOCATION: 4...4

(D) OTHER INFORMATION: phosphorylated tyrosine residue

(xi) SEQUENCE DESCRIPTION: SEQ ID NO:13:

Glu Pro Gln Tyr Glu Glu Ile Pro Ile Tyr Leu
1 5 10 15

(2) INFORMATION FOR SEQ ID NO:14:

(i) SEQUENCE CHARACTERISTICS:
(A) LENGTH: 19 amino acids
(B) TYPE: amino acid
(C) STRANDEDNESS: single
(D) TOPOLOGY: linear

(ii) MOLECULE TYPE: peptide
(iii) HYPOTHETICAL: NO
(iv) ANTISENSE: NO
(v) FRAGMENT TYPE: internal
(vi) ORIGINAL SOURCE:
(ix) FEATURE:

(A) NAME/KEY: Other
(B) LOCATION: 4...4
(D) OTHER INFORMATION: phosphorylated tyrosine residue

(xi) SEQUENCE DESCRIPTION: SEQ ID NO:14:

Asp Gly Gly Tyr Met Asp Met Ser Lys Asp Glu
1 5 10 15

(2) INFORMATION FOR SEQ ID NO:15:

(i) SEQUENCE CHARACTERISTICS:
(A) LENGTH: 19 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(ix) FEATURE:

(A) NAME/KEY: Other

(B) LOCATION: 6...6

(D) OTHER INFORMATION: phosphorylated tyrosine residue

(xi) SEQUENCE DESCRIPTION: SEQ ID NO:15:

Glu Asn Gly Leu Asn Tyr Ile Asp Leu Asp Leu
1 5 10 15

(2) INFORMATION FOR SEQ ID NO:16:

(i) SEQUENCE CHARACTERISTICS:

(A) LENGTH: 23 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(ix) FEATURE:

(A) NAME/KEY: Other
(B) LOCATION: 7...7
(D) OTHER INFORMATION: phosphorylated tyrosine residue

(xi) SEQUENCE DESCRIPTION: SEQ ID NO:16:

Thr Pro Pro His Leu Lys Tyr Phe Tyr Phe Val Val Ser Asp Ser
1 5 10 15
Gly
20

(2) INFORMATION FOR SEQ ID NO:17:

(i) SEQUENCE CHARACTERISTICS:
(A) LENGTH: 87 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:17:

TTCCATATGA AAAGTATTCG TATTCAGCGT GGCCCGGGCC GTCACCACCA CCACCACCAC
60
GGGATCCCCG CTGAAGAGTG GTACTTT
87

(2) INFORMATION FOR SEQ ID NO:18:

(i) SEQUENCE CHARACTERISTICS:

(A) LENGTH: 38 base pairs

(B) TYPE: nucleic acid

(C) STRANDEDNESS: single

(D) TOPOLOGY: linear

(ii) MOLECULE TYPE: cDNA

(iii) HYPOTHETICAL: NO

(iv) ANTISENSE: NO

(v) FRAGMENT TYPE:

(vi) ORIGINAL SOURCE:

(xi) SEQUENCE DESCRIPTION: SEQ ID NO:18:

GGAATTCTAG ATTACTAGGA CGTGGGGCAG ACGTT

38

(2) INFORMATION FOR SEQ ID NO:19:

(i) SEQUENCE CHARACTERISTICS:

(A) LENGTH: 46 base pairs

(B) TYPE: nucleic acid

(C) STRANDEDNESS: single

(D) TOPOLOGY: linear

(ii) MOLECULE TYPE: cDNA

(iii) HYPOTHETICAL: NO

(iv) ANTISENSE: NO

(v) FRAGMENT TYPE:

(vi) ORIGINAL SOURCE:

(xi) SEQUENCE DESCRIPTION: SEQ ID NO:19:

CGGGATCCTG GACGACGACG ACAAAGCTGA GGAGTGGTAT TTT

46

(2) INFORMATION FOR SEQ ID NO:20:

(i) SEQUENCE CHARACTERISTICS:

(A) LENGTH: 38 base pairs

(B) TYPE: nucleic acid

(C) STRANDEDNESS: single

(D) TOPOLOGY: linear

(ii) MOLECULE TYPE: cDNA

(iii) HYPOTHETICAL: NO

(iv) ANTISENSE: NO

(v) FRAGMENT TYPE:

(vi) ORIGINAL SOURCE:

(xi) SEQUENCE DESCRIPTION: SEQ ID NO:20:

GGAATTCTAG ACTATTAGGA CGTGGGGCAC ACGGT

38

(2) INFORMATION FOR SEQ ID NO:21:

(i) SEQUENCE CHARACTERISTICS:

(A) LENGTH: 48 base pairs

(B) TYPE: nucleic acid

(C) STRANDEDNESS: single

(D) TOPOLOGY: linear

(ii) MOLECULE TYPE: cDNA

(iii) HYPOTHETICAL: NO

(iv) ANTISENSE: NO

(v) FRAGMENT TYPE:

(vi) ORIGINAL SOURCE:

(xi) SEQUENCE DESCRIPTION: SEQ ID NO:21:

CGGGATCCTG GACGACGACG ACAAAGAGCC CGAACCCTGG TTCTT

48

(2) INFORMATION FOR SEQ ID NO:22:

(i) SEQUENCE CHARACTERISTICS:

(A) LENGTH: 35 base pairs

(B) TYPE: nucleic acid

(C) STRANDEDNESS: single

(D) TOPOLOGY: linear

(ii) MOLECULE TYPE: cDNA

(iii) HYPOTHETICAL: NO

(iv) ANTISENSE: NO

(v) FRAGMENT TYPE:

(vi) ORIGINAL SOURCE:

(xi) SEQUENCE DESCRIPTION: SEQ ID NO:22:

GCTCTAGACT ATTACTGGGG CTTCTGGGTC TG
35

(2) INFORMATION FOR SEQ ID NO:23:

(i) SEQUENCE CHARACTERISTICS:

(A) LENGTH: 46 base pairs

(B) TYPE: nucleic acid

(C) STRANDEDNESS: single

(D) TOPOLOGY: linear

(ii) MOLECULE TYPE: cDNA

(iii) HYPOTHETICAL: NO

(iv) ANTISENSE: NO

(v) FRAGMENT TYPE:

(vi) ORIGINAL SOURCE:

(xi) SEQUENCE DESCRIPTION: SEQ ID NO:23:

GAAGATCTTG GACGACGACG ACAAATCCCG TGGGTGGTTT CAC
46

(2) INFORMATION FOR SEQ ID NO:24:

(i) SEQUENCE CHARACTERISTICS:

(A) LENGTH: 35 base pairs

(B) TYPE: nucleic acid

(C) STRANDEDNESS: single

(D) TOPOLOGY: linear

(ii) MOLECULE TYPE: cDNA

(iii) HYPOTHETICAL: NO

(iv) ANTISENSE: NO

(v) FRAGMENT TYPE:

(vi) ORIGINAL SOURCE:

(xi) SEQUENCE DESCRIPTION: SEQ ID NO:24:

GCTCTAGACT ATTAACTAGT GGGATCGGAG CA

35

(2) INFORMATION FOR SEQ ID NO:25:

(i) SEQUENCE CHARACTERISTICS:

(A) LENGTH: 106 amino acids

(B) TYPE: amino acid

(C) STRANDEDNESS: single

(D) TOPOLOGY: linear

(ii) MOLECULE TYPE: peptide

(iii) HYPOTHETICAL: NO

(iv) ANTISENSE: NO

(v) FRAGMENT TYPE: internal

(vi) ORIGINAL SOURCE:

(xi) SEQUENCE DESCRIPTION: SEQ ID NO:25:

His Pro Trp Phe Phe Gly Lys Ile Pro Arg Ala Lys Ala Glu Glu Met
1 5 10 15

Leu Ser Lys Gln Arg His Asp Gly Ala Phe Leu Ile Arg Glu Ser Glu
20 25 30

Ser Ala Pro Gly Asp Phe Ser Leu Ser Val Lys Phe Gly Asn Asp Val
35 40 45

Gln His Phe Lys Val Leu Arg Asp Gly Ala Gly Lys Tyr Phe Leu Trp
50 55 60

Val Val Lys Phe Asn Ser Leu Asn Glu Leu Val Asp Tyr His Arg Ser
65 70 75 80

Thr Ser Val Ser Arg Asn Gln Gln Ile Phe Leu Arg Asp Ile Glu Gln
85 90 95

Val Pro Gln Gln Pro Thr Ile His Arg Asp
100 105

(2) INFORMATION FOR SEQ ID NO:26:

(i) SEQUENCE CHARACTERISTICS:
(A) LENGTH: 11 amino acids
(B) TYPE: amino acid
(C) STRANDEDNESS: single
(D) TOPOLOGY: linear

(ii) MOLECULE TYPE: peptide
(iii) HYPOTHETICAL: NO
(iv) ANTISENSE: NO
(v) FRAGMENT TYPE: internal
(vi) ORIGINAL SOURCE:
(ix) FEATURE:

(A) NAME/KEY: Other
(B) LOCATION: 6...6
(D) OTHER INFORMATION: phosphorylated tyrosine r
esidue

(xi) SEQUENCE DESCRIPTION: SEQ ID NO:26:

Leu Pro Val Pro Glu Tyr Ile Asn Gln Ser Val
1 5 10

## Description

BACKGROUND OF THE INVENTION

Mammalian bone is constantly undergoing bone remodeling, which is a dynamic process of bone resorption and bone formation. These processes are mediated by specialized cell types: bone formation is the result of the deposition of mineralized bone by osteoblast cells, and bone resorption is the result of the dissolution of bone matrix by osteoclast cells. Many bone diseases are brought about by an imbalance of bone formation relative to bone resorption. For instance, diseases such as osteoporosis and Paget's disease are characterized by a net loss of bone matrix. Thus, agents which inhibit bone resorption are useful for the treatment of such diseases.

An activated osteoclast resorbs bone by attaching to the bone matrix, and secreting proteolytic enzymes, organic acids and protons into the sealed compartment formed between its cell membrane and the bone matrix. The acidic environment and proteolytic enzymes effect the dissolution of bone in the sealed compartment to crest pits, or lacuna, in the bone surface, which are apparent when the osteoclast detaches from the bone.

A number of polypeptide growth factors and hormones mediate their cellular effects through a signal transduction pathway. Transduction of signals from the cell surface receptors for these ligands to intracellular effectors frequently involves phosphorylation or dephosphorylation of specific protein substrates by regulatory protein tyrosine kinases (PTK) and phosphatases. Tyrosine phosphorylation may be the primary, or possibly even the sole, indicator of signal transduction in multicellular organisms. Receptor-bound and intracellular PTKs regulate cell proliferation, cell differentiation and signalling processes in immune system cells.

Aberrant protein tyrosine kinase activity has been implicated or is suspected in a number of pathologies such as diabetes, atherosclerosis, psoriases, septic shock, bone loss, anemia, many cancers and other proliferative diseases. Accordingly, tyrosine kinases and the signal transduction pathways which they are part of are potential targets for drug design. For a review, see Levitzki et al. in Science 267, 1782-1788 (1995).

Many of the proteins comprising signal transduction pathways are present at low levels and often have opposing activities. The properties of these signalling molecules allow the cell to control transduction by means of the subcellular location and juxtaposition of effectors as well as by balancing activation with repression such that a small change in one pathway can achieve a switching effect.

The formation of transducing complexes by juxtaposition of the signalling molecules through protein-protein interactions are mediated by specific docking domain sequence motifs. Src homology 2 (SH2) domains, which are conserved non-catalytic sequences of approximately 100 amino acids found in a variety of signalling molecules such as non-receptor PTKs and kinase target effector molecules and in oncogenic proteins, play a critical role. The SH2 domains are highly specific for short phosphotyrosine-containing peptide sequences found in autophosphorylated PTK receptors or intracellular tyrosine kinases.

Approximately 60 proteins having distinct catalytic or other functional domains yet sharing conserved SH2 domains, conserved sequences of approximately 100 amino acids, have been identified. It is not known precisely which physiological responses in the body are controlled by each of these SH2 domains. Further, the SH2 domain-ligand/compound interactions are highly specific such that minor modifications in the structure of the ligand/compound will significantly alter the selectivity with which the ligand/compound binds to the various SH2 domains.

The consequences of non selective antagonism of SH2 domains can be quite severe. For example, although the src SH2 domain, the lck SH2 domain and the fyn SH2 domain are structurally similar, possessing a high degree of conservation between the domains, antagonism of the src SH2 domain is indicated herein as effecting bone resorption while antagonism of the lck SH2 domain or the fyn SH2 domain induces immunosuppression. The induction of immunosuppression would be undesirable in long term therapy for bone resorption disease.

Furthermore, it would be impractical to assay potential src SH2 domain antagonists in binding studies against all 60 known SH2 domains. Presently, there are no known compounds which selectively interact with the src SH2 domain.

It would be desirable to provide methods and compounds which allow the treatment of a bone resorption disease by antagonizing the src SH2 domain but which avoid the production of side effects observed in non-selective SH2 domain antagonists.

As disclosed herein it has unexpectedly been discovered that selective src SH2 domain antagonists can be identified by binding assays against the subset of SH2 domains consisting of; the src SH2 domain, the lck SH2 domain, the fyn SH2 domain, the SHPTP2 SH2 domain, the p85 domain, the Grb2 SH2 domain and the hcp SH2 domain.

From the binding information described hereinafter, it has unexpectedly been discovered that compounds which are specific for a human src SH2 domain with a binding affinity greater than fifty-fold higher than the binding affinity with which the compound binds to a human lck SH2 domain and a human fyn SH2 domain, and (b) binds to a human hcp SH2 domain, a human Grb2 SH2 domain, a human SH-PTP2 SH2 domain and a human p85 SH2 domain with a binding affinity which is greater than fifty-fold lower than the binding affinity with which the compound binds to such src SH2 domain are effective for treating a bone resorption disease.

## SUMMARY OF THE INVENTION

The present invention provides a method of treating a bone resorption disease in a subject which comprises administering to the subject a therapeutically effective amount of a compound which (a) binds to a human src SH2 domain with a binding affinity greater than fifty-fold higher than the binding affinity with which the compound binds to a human lck SH2 domain and a human fyn SH2 domain, and (b) binds to a human hcp SH2 domain, a human Grb2 domain, a human SH-PTP2 SH2 domain and a human p85 SH2 domain with a binding affinity which is greater than fifty-fold lower than the binding affinity with which the compound binds to such src SH2 domain.

The present invention also provides a method of treating osteoporosis in a subject which comprises administering to the subject a therapeutically effective amount of a compound which (a) binds to a human src SH2 domain with a binding affinity greater than fifty-fold higher than the binding affinity with which the compound binds to a human lck SH2 domain and a human fyn SH2 domain, and (b) binds to a human hcp SH2 domain, a human Grb2 domain, a human SH-PTP2 SH2 domain and a human p85 SH2 domain with a binding affinity which is greater than fifty-fold lower than the binding affinity with which the compound binds to such src SH2 domain.

The present invention also provides a method of impairing the function of osteoclasts in a subject which comprises administering to the subject an osteoclast function-inhibiting amount of a compound which (a) binds to a human src SH2 domain with a binding affinity greater than fifty-fold higher than the binding affinity with which the compound binds to a human lck SH2 domain and a human fyn SH2 domain, and (b) binds to a human hcp SH2 domain, a human Grb2 domain, a human SH-PTP2 SH2 domain and a human p85 SH2 domain with a binding affinity which is greater than fifty-fold lower than the binding affinity with which the compound binds to such src SH2 domain.

## DETAILED DESCRIPTION OF THE INVENTION

As used herein, the term "a bone resorption disease" means any disorder characterized by abnormal bone loss due to osteoclastic activity, preferably osteoporosis.

As used herein, the term "treating" and derivatives thereof means prophylactic or therapeutic therapy.

As used herein, the term "compound" means a nonpeptide chemical compound.

As used herein, unless other wise defined, the term " src SH2 domain antagonists" means a compound which (a) binds to a human src SH2 domain with a binding affinity greater than fifty-fold higher, preferably greater than one hundred-fold higher, than the binding affinity with which the compound binds to a human lck SH2 domain and a human fyn SH2 domain, and (b) binds to a human hcp SH2 domain, a human Grb2 SH2, a human SH-PTP2 SH2 domain and a human p85 SH2 domain with a binding affinity which is greater than fifty-fold lower, preferably greater than one hundred-fold lower, than the binding affinity with which the compound binds to such src SH2 domain.

The present invention provides a method of treating a bone resorption disease in a subject which comprises administering to the subject a therapeutically effective amount of a compound which (a) binds to a human src SH2 domain with a binding affinity greater than fifty-fold higher than the binding affinity with which the compound binds to a human lck SH2 domain and a human fyn SH2 domain, and (b) binds to a human hcp SH2 domain, a human Grb2 SH2, a human SH-PTP2 SH2 domain and a human p85 SH2 domain with a binding affinity which is greater than fifty-fold lower than the binding affinity with which the compound binds to such src SH2 domain.

A preferred aspect of the invention provides a method of treating a bone resorption disease in a subject which comprises administering to the subject a therapeutically effective amount of a compound which (a) binds to a human src SH2 domain with a binding affinity greater than fifty-fold higher than the binding affinity with which the compound binds to a human lck SH2 domain and a human fyn SH2 domain.

A preferred aspect of the invention provides a method of treating a bone resorption disease in a subject which comprises administering to the subject a therapeutically effective amount of a compound which (a) binds to a human src SH2 domain with a binding affinity greater than one hundred-fold higher than the binding affinity with which the compound binds to a human lck SH2 domain and a human fyn SH2 domain, and (b) binds to a human hcp SH2 domain, a human Grb2 SH2, a human SH-PTP2 SH2 domain and a human p85 SH2 domain with a binding affinity which is greater than one hundred-fold lower than the binding affinity with which the compound binds to such src SH2 domain.

A preferred aspect of the invention provides a method of treating a bone resorption disease in a subject which comprises administering to the subject a therapeutically effective amount of a compound which (a) binds to a human src SH2 domain with a binding affinity greater than one hundred-fold higher than the binding affinity with which the compound binds to a human lck SH2 domain and a human fyn SH2 domain.

The invention also provides a method of treating osteoporosis in a subject which comprises administering to the subject a therapeutically effective amount of a compound which (a) binds to a human src SH2 domain with a binding affinity greater than fifty-fold higher, preferably greater than one hundred-fold higher, than the binding affinity with which the compound binds to a human lck SH2 domain and a human fyn SH2 domain, and (b) binds to a human hcp SH2 domain, a human Grb2 domain, a human SH-PTP2 SH2 domain and a human p85 SH2 domain with a binding affinity

which is greater than fifty-fold lower, preferably greater than one hundred-fold lower, than the binding affinity with which the compound binds to such src SH2 domain.

A preferred aspect of the invention provides a method of treating osteoporosis in a subject which comprises administering to the subject a therapeutically effective amount of a compound which (a) binds to a human src SH2 domain with a binding affinity greater than fifty-fold higher, preferably greater than one hundred-fold higher, than the binding affinity with which the compound binds to a human lck SH2 domain and a human fyn SH2 domain.

The invention also provides a method of impairing the function of osteoclasts in a subject which comprises administering to the subject an osteoclast function-inhibiting amount of a compound which (a) binds to a human src SH2 domain with a binding affinity greater than fifty-fold higher, preferably greater than one hundred-fold higher, than the binding affinity with which the compound binds to a human lck SH2 domain and a human fyn SH2 domain, and (b) binds to a human hcp SH2 domain, a human Grb2 domain, a human SH-PTP2 SH2 domain and a human p85 SH2 domain with a binding affinity which is greater than fifty-fold lower, preferably greater than one hundred-fold lower, than the binding affinity with which the compound binds to such src SH2 domain.

A preferred aspect of the invention provides a method of impairing the function of osteoclasts in a subject which comprises administering to the subject a therapeutically effective amount of a compound which (a) binds to a human src SH2 domain with a binding affinity greater than fifty-fold higher, preferably greater than one hundred-fold higher, than the binding affinity with which the compound binds to a human lck SH2 domain and a human fyn SH2 domain.

The inhibitory activity of compounds at the different human SH2 domains was determined in vitro using SH2 domains expressed as fusion proteins in E. coli as further described in detail in Example 11 below.

The data shown in the accompanying Tables 1 and 2 and in Example 12 indicate that src SH2 domain antagonists have significant efficacy in the fetal rat long bone (FRLB) assay. This in vitro activity is recognized in the art as correlating with efficacy in treating a bone resorption disease in vivo. This in vitro activity is also recognized in the art as correlating with efficacy in impairing the function of osteoclasts in vivo.

The present invention therefore provides a method of treating a bone resorption disease, which comprises administering a quantity of a src SH2 domain antagonists defined as herein in a quantity effective to inhibit bone resorption. The drug may be administered to a patient afflicted with a bone resorption disease or in danger of contracting a bone resorption disease by any conventional route of administration, including, but not limited to, intravenous, intramuscular, oral, subcutaneous, intradermal, and parenteral. The quantity effective to inhibit bone resorption is from about 0.001 mg per kg to about 10.0 mg per kg of subject body weight. The selected dose will be an efficacious, nontoxic quantity selected from about 0.001 mg per kg to about 10.0 mg per kg of subject body weight. The selected dose will be administered from about 1-6 times daily.

The method of treating a bone resorption disease disclosed in the present invention may also be carried out using a pharmaceutical composition comprising an src SH2 domain antagonists defined herein and a pharmaceutically acceptable carrier. The composition may contain between 0.05 mg and 500 mg of a src SH2 domain antagonist, and may be constituted into any form suitable for the mode of administration selected. Compositions suitable for oral administration include solid forms, such as pills, capsules, granules, tablets, and powders, and liquid forms, such as solutions, syrups, elixers, and suspensions. Forms useful for parenteral administration include sterile solutions, emulsions, and suspensions.

The present invention further provides a method of impairing the function of osteoclasts, which comprises administering a quantity of a src SH2 domain antagonists defined as herein in a quantity effective to inhibit bone resorption. The drug may be administered to a patient afflicted with a bone resorption disease or in danger of contracting a bone resorption disease by any conventional route of administration, including, but not limited to, intravenous, intramuscular, oral, subcutaneous, intradermal, and parenteral. The quantity effective to impair osteoclasts function is from about 0.001 mg per kg to about 10.0 mg per kg of subject body weight. The selected dose will be an efficacious, nontoxic quantity selected from about 0.001 mg per kg to about 10.0 mg per kg of subject body weight. The selected dose will be administered from about 1-6 times daily.

The method of impairing the function of osteoclasts disclosed in the present invention may also be carried out using a pharmaceutical composition comprising an src SH2 domain antagonists defined herein and a pharmaceutically acceptable carrier. The composition may contain between 0.05 mg and 500 mg of a src SH2 domain antagonist, and may be constituted into any form suitable for the mode of administration selected. Compositions suitable for oral administration include solid forms, such as pills, capsules, granules, tablets, and powders, and liquid forms, such as solutions, syrups, elixers, and suspensions. Forms useful for parenteral administration include sterile solutions, emulsions, and suspensions.

The drug may otherwise be prepared as a sterile solid composition which may be dissolved or suspended at the time of administration using sterile water, saline, or other appropriate sterile injectable medium. Carriers are intended to include necessary and inert binders, suspending agents, lubricants, flavorants, sweeteners, preservatives, dyes and coatings.

Optimal dosages to be administered may be readily determined by those skilled in the art, and will vary with the

particular src SH2 domain antagonist in use, the strength of the preparation, the mode of administration, and the advancement of the disease condition. Additional factors depending on the particular patient being treated will result in a need to adjust dosages, including patient age, weight, diet, and time of administration.

The invention also provides for the use of a src SH2 domain antagonists in the manufacture of a medicament for use in the treatment of a bone resorption disease.

The invention also provides for the use of a src SH2 domain antagonists in the manufacture of a medicament for use in the treating osteoporosis.

The invention also provides for the use of a src SH2 domain antagonists in the manufacture of a medicament for use in inhibiting osteoclast function.

The invention also provides for a pharmaceutical composition for use in the treatment of a bone resorption disease which comprises a src SH2 domain antagonists.

The invention also provides for a pharmaceutical composition for use in the treatment of osteoporosis which comprises a src SH2 domain antagonists.

The invention also provides for a pharmaceutical composition for use in inhibiting osteoclast function which comprises a src SH2 domain antagonists.

No unacceptable toxicological effects are expected when the methods of the invention are utilized in accordance with the present invention.

Without further elaboration, it is believed that one skilled in the art can, using the preceding description, utilize the present invention to its fullest extent. The following Examples are, therefore, to be construed as merely illustrative and not a limitation of the scope of the present invention in any way.

Experimental Details

As used herein, unless otherwise indicated, the symbol ° means °C.

L-3,5-Dibromotyrosine can be prepared by methods known in the art, for example as described in "Thyoid Hormones and Analogues. I. Synthesis, Physical Properties and Theoretical Calculations" E. C. Jorgensen, Hormonal Proteins and Peptides, Vol. VI, 1978, Academic Press, N.Y. and references cited therein.

L-3,5-dibromo-N-trifluoroacetyl-tyrosine methyl ester (for use in Example 2 (e) and in Example 2B (b)) can be prepared according to the following procedure.

L-3,5-Dibromotyrosine (500 g) was suspended in methanol (5 liters) and dry hydrogen chloride passed through the stirred suspension for 5 hours. The reaction mixture was evaporated to dryness, the residue suspended in water (4 liters), and the pH adjusted to 6 with 40% sodium hydroxide. The precipitate was collected and washed with water to give L-3,5-dibromotyrosine methyl ester (467 g, 90%), m.p. 201°-203°. The ester (768 g) was suspended in chloroform (2.7 liters) and ethyl acetate (2.7 liters), then trifluoroacetic anhydride (565 g) was added over 0.5 hour, keeping the temperature below 35°. The mixture was left overnight, then water (2 liters) was added and the pH adjusted to 7 by the addition of saturated sodium bicarbonate solution. The organic layer was removed, washed with water, dried with anhydrous magnesium sulphate and evaporated. The residue was recrystallised from aqueous methanol to give L-3,5-dibromo-N-trifluoroacetyl-tyrosine methyl ester (786 g, 81%), m.p. 136°-7°.

## Scheme 1 as used in Example 6 below

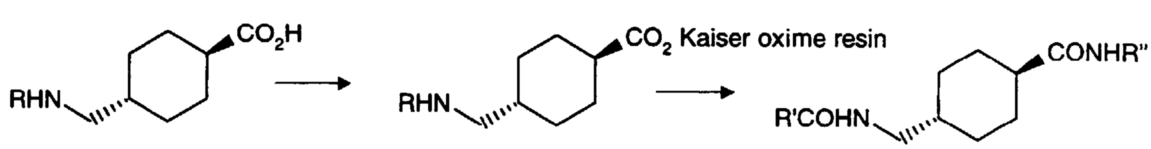

1: R=H
2: R=Boc

3: R=Boc
4: R=H
5: R= COR'

6

The amino group of 4-trans-aminomethyl-cyclohexyl-carboxylic acid **1** is protected with a standard protective group such as with a Boc group (Boc anhydride, NaOH, $H_2O$, dioxane) to form **2**, then is coupled to Kaiser oxime resin (Kaiser, E.T.; *et al J Am Chem Soc* **1985**, *107,* 7087-7092) using a coupling reagent such as DCC to form **3**. The amine is then deprotected under standard conditions (25% TFA, methylene chloride) to form **4**, then is acylated with standard conditions (such as with HBTU, NMM in DMF or DCC or DIC in DMF or NMP) to form **5**. The compound is then cleaved from the resin with various amines to form the final desired product **6**.

Compounds 1 to 10 are prepared according to Examples 1 to 10 which follow.

Example 1

Preparation of 7-[D,L-α-Amino-α-(4-carboxyphenyl)acetamido]-3-[2-(5-methyl-1,3,4-thiadiazolyl)thiomethyl]Δ$^3$-cephem-4-carboxylic Acid (Compound 1)

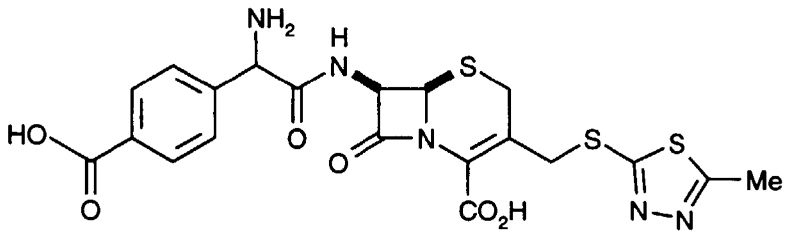

a) 4-Hydroxymethylbenzaldehyde

To a solution of 1,4-benzenedicarboxaldehyde (50.0 g, 0.373 mole) in dry tetrahydrofuran (200 mL) under nitrogen in an ice bath was added dropwise lithium tri(tert-butoxy)aluminum hydride (104.0 g, 0.410 mole) in 500 mL of tetrahydrofuran. After stirring for one half hour in an ice bath, the reaction mixture was poured into 2 L of ice cold 2 N hydrochloric acid. The aqueous solution was extracted with four 800 mL portions of ether. The combined ether layers were washed with sodium bicarbonate solution, brine and dried. Evaporation of the solvent afforded 46 g of crude material that was purified by chromatography (alumina, ether elution) to provide the title compound as a crystalline material (17.6 g, 35%): mp 44.5-46 °C

b) 5-(4-Hydroxymethyphenyl)hydantoin

To a stirred mixture of 4-hydroxymethylbenzaldehyde (10.0 g, 73.5 mmol) and ammonium carbonate (17.1 g, 150 mmol) in 110 mL of 60% aqueous ethanol heated to 50 °C there was added sodium cyanide (4.0 g, 81 mmol) in 10 mL of water. The mixture was stirred and heated at 50-60 °C for 3 h and then at 85 °C for one hour. After cooling in an ice bath, the pH of the solution was adjusted to 6 by addition of concentrated hydrochloric acid. Upon overnight cooling, the solid which had precipitated was filtered, washed with water and dried to provide the title compound (11.0 g, 72%): mp 189-196 °C.

c) 4-Hydroxymethyphenylglycine

A mixture of compound of Example 1(b) (10.9 g, 53 mmol) and barium hydroxide octahydrate (25.5 g, 81 mmol) in 125 mL of water was stirred under reflux for 18 h. The reaction mixture was cooled and acidified to pH 1 with concentrated sulfuric acid; the barium sulfate was filtered and the pH of the filtrate brought to 6 with lead carbonate. After filtration of the lead sulfate, the filtrate was saturated with hydrogen sulfide and the lead sulfide filtered. The aqueous solution was then concentrated to 100 mL by azeotroping with ethanol under reduced pressure to provide, after cooling, the title compound (5.2 g, 54%): mp 230-231 °C.

d) N-*tert*-Butoxycarbonyl-4-hydroxymethyphenylglycine

To a solution of 4-hydroxymethyphenylglycine (8.0 g, 44 mmol) and triethylamine (8.8 g, 87 mmol) in 160 mL of water was added *tert*-butoxycarbonyl azide (6.95 g, 49 mmol) in 120 mL of tetrahydrofuran. After stirring overnight at room temperature, the reaction mixture was washed twice with 200 mL potions of ether. The aqueous layer was covered with ether and acidified to pH 3-3.5 with 3 N hydrochloric acid in an ice bath. The acidic solution was extracted with ether and the combined organic extrats washed with brine, dried and evaporated. The resulting oil was triturated with chloroform-hexane and the solid filtered off to provide the title compound (7.7 g, 63%): mp 139-141.5 °C.

)N-*tert*-Butoxycarbonyl-4-hydroxymethyphenylglycine Methyl Ester

To a solution of compound of Example 1(e) (5.6 g, 20 mmol) was added dimethyl sulfate (3.1 g, 24 mmol) and diisopropyl amine (5.2 g, 40 mmol) in methanol (10 mL). The mixture was refluxed for 20 min and was then treated with 2 N aqueous hydrochloric acid. The aqueous solutin was extracted with ethyl acetate three times and the combined organic extracts washed with 5% aqueous sodium bicarbonate and brine. Evaporation of the solvent provided the title

compound as an oil (3.2 g, 55%).

f) N-*tert*-Butoxycarbonyl-4-carboxyphenylglycine Methyl Ester

A solution of the compound of Example 1(e) (0.62 g, 2.1 mmol) in 50 ml of acetone was treated with excess Jones reagent (8N chromic acid) at 25° C. The reaction mixture was stirred at room temperature for 2 hours. The green solid was filtered off and excess CrO3 was decomposed by isopropyl alcohol. The filtrate was dried over anhydrous sodium sulfate and treated with activated charcoal. Solid was filtered off and the filtrate was evaporated to dryness to yield 0.38 g of title compound as white solid: mp 126-128 °C.

g) 1,1-Dimethylethyl N,N'-Bis(1-methylethyl)carbamimidate

The title compound was prepared by reaction of neat N,N'-diisopropylcarbodiimide (1.0 equiv) with 2-methyl-2-propanol (1.15 equiv) in the presence of Cucl (0.01 equiv) for 1 day at room temperature, according to the procedure of Santini et al. *( J. Org. Chem.* **1994**, 59, 2261).

h) N-*tert*-Butoxycarbonyl-4-(*tert*-butoxycarbonyl)phenylglycine Methyl Ester

A solution of the compound of Example 1(f) (1.0 g, 3.2 mmol) and 1,1-dimethylethyl N,N'-bis(1-methylethyl)carbamimidate (1.3 mg, 6.5 mmol) of in dry dichloromethane was stirred at room temperature over night. Di-isopropylurea was filtered off and the excess 1,1-dimethylethyl N,N'-bis(1-methylethyl)carbamimidate was decomposed with water. Layers were separated and the dichloromethane solution was washed with 5% aqueous sodium bicarbonate and brine and dried over anhydrous sodium sulfate. Solvent was evaporated off and the residue was treated with diethyl ether. Additional di-isopropylurea was filtered off and the organic filtrate was evaporated to yield the title compound as an oil (870 mg, 74%).

i) N-*tert*-Butoxycarbonyl-4-(*tert*-butoxycarbonyl)phenylglycine

A solution of the compound of Example 1(h) (760 mg, 2.1 mmol) in 18 mL of 5% aqueous sodium bicarbonate, 18 mL of 5% aqueous sodium carbonate and 36 mL of methanol was stirred ovenight at room temperature for 5 hours. The reaction mixture was diluted with water, washed with ethyl acetate and the aqueous solution was covered with fresh ethyl acetate and acidified to pH 2 with 3N HCl. Layers were separated and the aqueous solution was extraced with ethyl acetate 2 more times. Ethyl acetate solutions were dried over anhydrous sodium sulfate and evaporated to yield title compound as a white solid (600 mg, 82%): mp 77-79 °C.

j) *tert*-Butyl 7-Amino-3-[2-(5-methyl-1,3,4-thiadiazolyl)thiomethyl]$\Delta^3$-cephem-4-carboxylate.

A solution of *tert*-butyl 7-aminocephalosporanate (prepared from 7-aminocephalosporanic acid by reaction with isobutylene and sulfuric acid in 1,2-dimethoxyethane, according to the procedure of Blacklock et al., *J. Org. Chem.* **1989**, *54*, 3907), sodium bicarbonate and 2-mercapto-5-methyl-1,3,4-thiadiazole in phosphate buffer (pH 6.4) is stirred for 6 h at 60 °C. The reaction mixture is worked up by extraction with aqueous hydrochloric acid/ethyl acetate to provide the title compound.

k) *tert*-Butyl 7-[D,L-$\alpha$-(*tert*-Butoxycarbonylamino)-$\alpha$-[4-(*tert*-butoxycarbonyl)phenyl]]acetamido-3-[2-(5-methyl-1,3,4-thiadiazolyl)thiomethyl]$\Delta^3$-cephem-4-carboxylate

A mixture of N-*tert*-butoxycarbonyl(4-(*tert*-butoxycarbonyl)phenylglycine of Example 1(i) (351 mg, 1 mmol), *tert*-butyl 7-amino-3-[2-(5-methyl-1,3,4-thiadiazolyl)thiomethyl]$\Delta^3$-cephem-4-carboxylate of Example 1(j) (368 mg, 1 mmol) and DCC (212 mg, 1 mmol) in dry dichloromethane was stirred at room temperature for 3 hours. The dicyclohexylurea was filtered off and the filtrate was evaporated to dryness. The residue was dissolved in ethyl acetate and the ethyl acetate solution was washed with 5% aqueous sodium bicarbonate, 2.5% sulfuric acid, 5% aqueous sodium bicarbonate, brine and dried over anhydrous sodium sulfate. The solvent was evaporated to yield 0.6 g of crude product. Purification by silica gel chromatography (elution with 30:70 ethyl acetate / benzene) provided the title compound (430 mg, 61%): mp 110-112 °C.

l) 7-[D,L-α-Amino-α-(4-carboxyphenyl)acetamido]-3-[2-(5-methyl-1,3,4-thiadiazolyl)thiomethyl]$\Delta^3$-cephem-4-carboxylic Acid

A solution of the compound of Example 1(k) (400 mg, 0.57 mmol) was stirred in 7.2 mL of trifluoroacetic acid and 0.8 mL of thiophenol. The reaction mixture was stirred at 0 °C for 30 minutes and at room temperature for 1 hour. The solvents were evaporated off in a 40° C water bath and the residue was triturated with diethyl ether three times; the solid product was dissolved in small amounts of methanol and the product was precipitated by addition of diethyl ether to afford the title compound (300 mg): mp 170-175 °C.

Example 2

Preparation of L-3,5-Dibromo-3'-(6-oxo-3(1H)-pyridazinylmethyl)-thyronine (Compound 2)

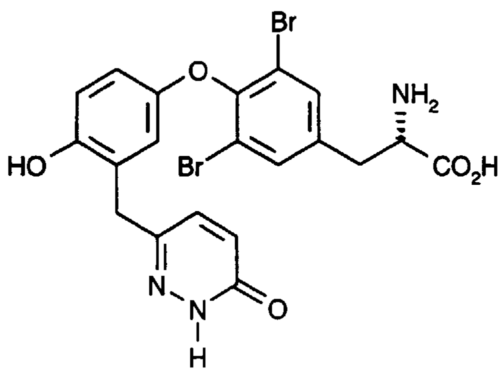

(a) o-Methoxyphenylacetonitrile (23.64 g) and 3,6-dichloropyridazine (23.93 g) were dissolved in dry dimethylformamide (50 ml) and sodium hydride (16.23 g of a 50% dispersion in oil) was slowly added in portions to the stirred solution over 2 hours. The mixture was poured on to excess crushed ice and extracted with dichloromethane. The organic layer was removed and washed with water, dried with anhydrous magnesium sulphate, charcoaled and evaporated to dryness. The residue crystallised from dichloromethane/petroleum spirit to give 1-(6-chloro-3-pyridazinyl)-1-(2-methoxyphenyl)-acetonitrile (35.5 g 85%), m.p. 91°-92°.

(b) This nitrile (33.5 g) was dissolved in concentrated hydrochloric acid (200 ml), acetic acid (100 ml) and water (100 ml) and the solution refluxed with stirring. After 6 hours the solvents were evaporated and the residue recrystallised from ethyl acetate/petroleum spirit to give 2-(6-oxo-3(1H)-pyridazinylmethyl)anisole (21.4 g, 77%), m.p. 142°-3°.

(c) This pyridazinone (15.7 g) was dissolved in phosphorous oxychloride (22 ml) and the solution heated with stirring at 55° (oil bath) for 1 hour. The cooled mixture was slowly poured onto crushed ice, and extracted with dichloromethane. The organic layer was separated and washed with saturated sodium bicarbonate solution, dried with anhydrous magnesium sulphate and evaporated. The residue was combined with a smaller batch (from 2.16 g of the pyridazinone) and extracted several times with boiling petroleum spirit (60°-80°). The combined extracts were charcoaled and evaporated to give 2-(6-chloro-3-pyridazinylmethyl)-anisole (16.95 g, 87%), m.p. 63°.

(d) To a stirred suspension of iodine tristrifluoroacetate (prepared by treatment of iodine (2.54 g) with fuming nitric acid (5 ml) in acetic anhydride and trifluoroacetic acid) in trifluoroacetic anhydride (25 ml) at -15° was added the above chloropyridazine (9.39 g) in trifluoroacetic acid (20 ml) and trifluoroacetic anhydride (25 ml), keeping the temperature below -15°. The mixture was stirred at room temperature overnight, concentrated, then a solution of sodium acetate (25 g) and sodium perchlorate (15 g) in water (200 ml) was added. The mixture was extracted with chloroform, the organic solution dried with anhydrous magnesium sulphate, then concentrated to 50 ml and poured into stirred ether (250 ml). The precipitate was collected and dried to give crude 4,4'-dimethoxy-3,3'-bis-(6-chloro-3-pyridazinyl-methyl)-diphenyl iodonium perchlorate (14 g). $^1H$ NMR δ(DMSO-$d_6$) 3.80 (3H, s, -OC$\underline{H}_3$), 4.20 (2H, s, -C$\underline{H}_2$Ar), 7.05 (1H, m, Ar-5$\underline{H}$), 7.65 (2H, m, Py$\underline{H}$) and 8.00 (2H, m, Ar-2,6$\underline{H}$).

(e) The above iodonium salt (12.45 g), L-3,5-dibromo-N-trifluoroacetyl tyrosine methyl ester (8.98 g), triethylamine (4.05 g) and copper bronze (1.0 g) were stirred in dichloromethane (50 ml) for 18 hours. The mixture was filtered, washed with aqueous acetic acid, 2N sodium hydroxide, then water, then dried with anhydrous magnesium sulphate and evaporated. The residue was combined with a smaller batch (from 0.72 g of the iodonium salt) and purified by column chromatography on silica gel (400 g). Elution with ethyl acetate/petroleum spirit (60°-80°) [1:3] gave L-3,5-dibromo-3'-(6-chloro-3-pyridazinylmethyl)-O-methyl-N-trifluoroacetyl-1-thyronine methyl ester (4.0 g)

as a tan coloured froth. $^1H$ NMR $\delta(CDCl_3)$ 3.06 (2H, m, ArC$\underline{H}_2$CH), 3.84 and 3.93(6H, 2s, -OC$\underline{H}_3$), 4.19(2H, s, ArC$\underline{H}_2$Py), 4.75(1H, m, ArCH$_2$C$\underline{H}$), 6.62(3H, m, ArH), 7.17(2H, m, Py$\underline{H}$) and 7.23(2H, s, Ar$\underline{H}$).

(f) The above dibromo compound (3.27 g) was dissolved in acetic acid (20 ml) containing sodium acetate (0.79 g). The solution was refluxed for 1.25 hours, sufficient water (approximately 2 ml) added to dissolve the precipitated sodium chloride, and the solution evaporated to dryness. The residue was partitioned between water and ethyl acetate, the organic layer removed and washed with saturated sodium bicarbonate, then dried with anhydrous magnesium sulphate and evaporated to dryness. The residue was crystallised from ethyl acetate/petroleum spirit (60°-80°) to give L-3,5-dibromo-O-methyl-3'-(6-oxo-3(1H)pyridazinylmethyl)-N-trifluoro-acetylthyronine methyl ester (2.52 g, 79%), m.p. 176°-8°.

(g) This pyridazinone (2.45 g) was dissolved in dry dichloromethane (40 ml) and cooled with stirring at 0°. Boron tribromide (6.46 g) in dichloromethane (3 ml) was added. A red-brown precipitate formed. The mixture was stirred at room temperature for 1.5 hours, then crushed ice was added. The mixture was filtered, the precipitate collected and dissolved in 2N sodium hydroxide (30 ml). The solution was heated on a steam bath for 15 minutes, acetic acid was then added to pH5, and the mixture cooled. The resulting precipitate was collected, washed and dried to give L-3,5-dibromo-3'-(6-oxo-3(1H)-pyridazinylmethyl)-thyronine (1.74 g, 88%), m.p. 278°-9° (dec.).

Alternatively, instead of using the perchlorate salt prepared in (d) for reaction step (e), the iodonium trifluoroacetate salt can be used which is prepared as follows: Iodine (159 g) was suspended in trifluoroacetic anhydride (1 liter) and stirred under nitrogen whilst fuming nitric acid (350 ml) was added over 1.5 hours, keeping the temperature between 36° and 40°. Trifluoroacetic anhydride (300 ml) was then added and the mixture maintained at 40° under a stream of nitrogen until all nitrogen oxides were removed, then allowed to stand at room temperature overnight. The solvent was then removed under reduced pressure and the residual solvent removed by azeotroping with trifluoroacetic anhydride (2 X 300 ml). The pale yellow residual solid was then suspended in trifluoroacetic anhydride (1.2 liters) with stirring and was cooled to -20°. A solution of 2-(6-chloro-3-pyridazinylmethyl)anisole (600 g) in triflouoracetic acid (1.2 liters) was then added dropwise, maintaining the temperature between -10° and -20°. The mixture was stirred at -10° for 1 hour and at room temperature overnight, then the solvent removed under reduced pressure and the residue poured into a solution of sodium sulphate (3.5 kg) in water (20 liters) with stirring. The pH of this mixture was adjusted to approximately pH 2 using dilute aqueous sodium hydroxide, then extracted with dichloromethane (2 X 3 liters, 1 x 2 liters), the organic extracts combined, dried ($MgSO_4$), filtered, and reduced in volume to 2 liters, then added to vigorously stirred diethyl ether (12 liters). The dark grey precipitated solid was filtered off, washed with ether, and dried in a vacuum oven at 40° for 6 hours to give 4,4'-dimethoxy-3,3'-bis-(6-chloro-3-pyridazinylmethyl) diphenyl iodonium trifluoroacetate (8.14 g, 90%), m.p. 145°-147°.

Further reaction of this salt using procedures analogous to those described in 2(e), (f) and (g) above gives the required L-3,5-dibromo-3'-(6-oxo-3(1H)-pyridazinylmethyl)thyronine.

Example 2A

Preparation of L-3,5-Dibromo-3'-(6-oxo-3(1H)-pyridazinylmethyl)thyronine (Compound 2)

(a) 2-(6-Chloro-3-pyridazinylmethyl)anisole (prepared as described in Example 2(c)(2.35 g) was dissolved in dry dichloromethane (20 ml) and cooled with stirring to -50°. Boron tribromide (3 ml) was then added dropwise, and the solution was allowed to warm to room temperature. After 0.5 hours the orange reaction mixture was poured into ice/water (200 ml) and acetone added to dissolve the precipitated solid. The mixture was extracted with dichloromethane, the organic extracts were separated, washed with water, dried, and evaporated. The residue was recrystallised from ethyl acetate and petroleum spirit to give 2-(6-chloro-3-pyridazinylmethyl)-phenol (1.75 g, 80%), m.p. 132°-132.5°. Anal. Found: C, 59.61; H, 4.13; N, 12.47; Cl, 16.09; $C_{11}H_9ClN_2O$ Requires: C, 59.87; H, 4.11; N, 12.70; Cl, 16.07%.

(b) To a stirred solution of this phenol (2.4 g) and urea (14 g) in 75% aqueous sulphuric acid (100 ml) t-butanol (17 ml) was added slowly. The mixture was stirred well and further quantities of t-butanol were added after 4 hours (18 ml), 24 hours (5 ml), and 28 hours (20 ml). After 120 hours the mixture was poured into water, the organic phase separated and discarded and the aqueous phase extracted thoroughly with ether. The combined ether extracts were washed with saturated brine, then dried and evaporated. The residue was recrystallised from ether and petroleum spirit to give 2,4-di-t-butyl-6-(6-chloro-3-pyridazinylmethyl)phenol (3.43 g, 94%), m.p. 143.0°-143.5°. Anal. Found: C, 68.32; H, 7.51; N, 8.36; Cl, 10.89; $C_{19}H_{25}ClN_2O$. Requires: C, 68.56; H, 7.57; N, 8.41; Cl, 10.65%).

(c) A solution of this phenol (1.95 g), L-3,5-dibromo-N-trifluoroacetyl tyrosine methyl ester (3.24 g) in dietheyl ether (100 ml) was stirred under argon at room temperature and then treated with active manganese dioxide (3 X 5 g). After 4 hours the mixture was filtered, and titanium tetrachloride (5 ml) added. After 2 minutes the dark solution

was treated with water and extracted well with ethyl acetate. The organic extracts were combined, washed with saturated brine, dried and evaporated. The residue was chromatographed on silica gel with petroleum spirit and ether as eluant to give L-3,5-dibromo-5'-t-butyl-3'-(6-chloro-3-pyridazinylmethyl)-N-trifluoroacetyl thyronine methyl ester (2.31 g, 55%), m.p. 84°-86°.

(d) A solution of this dibromothyronine (2.76 g) and anhydrous sodium acetate (0.78 g) in acetic acid (25 ml) was heated at reflux for 10 hours, then cooled and poured into ice-water. The precipitated solid was filtered off, dissolved in ethyl acetate, dried, and evaporated to give L-3,5-dibromo-5'-t-butyl-3'-(6-oxo-3(1H)-pyridazinylmethyl)-N-trifluoroacetylthyronine methyl ester, (2.4 g, 55%), m.p. 112°-115°.

(e) A solution of this pyridazinone (0.200 g) and Hbr (1 ml) in glacial acetic acid (20 ml) was heated at reflux for three days. The solution was then cooled, diluted with water, basified with aqueous 2N sodium hydroxide solution and brought to pH 6 by addition of acetic acid. The precipitated solid was filtered, washed, and dried to give L-3,5-dibromo-3'-(6-oxo-3(1H)-pyridazinylmethyl)thyronine (0.100 g, 65%), m.p. 245°-247° (dec.), spectroscopically identical with that previously isolated (Example 2(g)).

Example 2B

Preparation of L-3,5-Dibromo-3'-(6-oxo-3(1H-pyridazinylmethyl)-thyronine (Compound 2)

(a) To a solution of iodine tristrifluoroacetate (prepared by treatment of iodine (10.0 g) with fuming nitric acid (20.95 ml) in acetic anhydride and trifluoroacetic acid) in acetic anhydride (50 ml), cooled to -10°, was added dropwise a solution of 2-methoxybenzyl cyanide (30.0 g) in trifluoroacetic acid (60 ml) and acetic anhydride (30 ml). The temperature of the mixture was maintained below 0° during the addition then allowed to stand at room temperature overnight. The mixture was then poured into a well-stirred ice-cold solution of sodium acetate (100 g) and sodium perchlorate (13.0 g) in water (600 ml). The solid which precipitated was filtered off, washed with water and diethyl ether to give 3,3'-dicyanomethyl-4,4'-dimethoxy-diphenyl iodonium perchlorate as a fine buff solid (23.6 g, 57%), m.p. 183°-4° (from methanol/diethyl ether).

(b) A solution of this iodonium salt (22.6 g), L-3,5-dibromo-N-trifluoroacetyl-tyrosine methyl ester, triethylamine (6.1 g) in dichloromethane (300 ml) was treated with copper bronze (1 g) and the mixture stirred at room temperature for 20 hours. The mixture was then filtered and the filtrate washed with 2N aqueous hydrochloric acid ( 2 X 200 ml), water (2 X 200 ml), and 2N aqueous sodium hydroxide solution (3 X 200 ml), then the organic solution was dried over magnesium sulphate and evaporated under reduced pressure. The oily residue was dissolved in dichloromethane (30 ml) and poured into petroleum spirit. A solid precipitated which was filtered off and recrystallised from dichloromethane/petroleum spirit to give L-3,5-dibromo-3'-cyanomethyl-O-methyl-N-trifluoroacetylthyronine methyl ester as a colourless crystalline solid, m.p. 148°-149°. The mother liquors were chromatographed on silica gel to give further quantities of this compound (total = 8.05 g, 31%).

(c) To a solution of this dibromothyronine (120 mg) and 3,6-dichloropyridazine (31 mg) in dry dimethylformamide (2 ml), sodium hydride (30 mg of a 50% suspension in oil) was added and the reaction mixture allowed to stand at room temperature for 50 min. It was then treated with ice, and the aqueous mixture extracted with dichloromethane, the organic solution washed with saturated brine, then dried and evaporated. The residue was chromatographed on a preparative silica gel chromatography plate from which 3,5-dibromo-3'-(1-(6-chloro-3-pyridazinyl)-1-cyanomethyl)-O-methyl-N-trifluoroacetylthyronine methyl ester (5 mg) was isolated. $^1$H NMR $\delta$($CDCl_3$) 3.12 (1H, m), 3.27 (1H, m), 3.79 (3H, s), 3.86 (3H, s), 4.86 (1H, m), 5.80 (1H, s), 6.72 (1H, dd), 6.83 (1H, d), 7.04 (1H, d), 7.15 (1H, broad m), 7.37 (2H, s), 7.50 (2H, dd).

Elaboration of this intermediate by standard methods gives the title compound.

Example 3

Preparation of 8,8-Ethylenedioxy-2,3,7,8,9,10-hexahydro-4-methyl-1H-benzo[b]thieno[2,3-b]pyrazolo[3,4-d]pyridin-3-one (Compound 3)

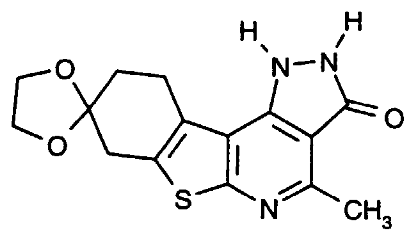

a) Ethyl 2-Cyano-2-(4,4,-ethylenedioxycyclohexylidene)acetate

To a mixture of 1,4 cyclohexanedione monoethylene ketal (25 g, 0.160 mol) and ethyl cyanoacetate (18 g, 0.160 mol) in toluene (400 mL) was added dropwise diethylamine (25 g, 0.337 mol) at room temperature. The reaction mixture was heated at reflux overnight (using a Dean Stark apparatus). The mixture was cooled and partitioned with ethyl acetate and saturated aqueous sodium bicarbonate (3x). The organic extracts were dried over sodium sulfate, filtered, concentrated in vacuo and recrystallized from ethanol to yield the title compound as an white solid (15.8 g, 45%): mp 80-81 °C; $^1H$ NMR (400 MHz, $CDCl_3$) δ 4.28 (q, $J$ = 7.2 Hz, 2 H), 4.00 (s, 4 H), 3.18 (t, $J$ = 6.5 Hz, 2 H), 2.85 (t, $J$ = 6.5 Hz, 2 H), 1.89 (t, $J$ = 6.5 Hz, 2 H), 1.82 (t, $J$ = 6.5 Hz, 2 H), 1.35 (t, $J$ = 7.1 Hz, 3 H).

b) Ethyl 2-Amino-6,6-ethylenedioxy-4,5,6,7-tetrahydrobenzo[b]thiophene-3-carboxylate

To a suspension of compound of Example 3(a) (10 g, 45.6 mmol), sulfur (1.6 g, 50.2 mmol) in ethanol (164 mL) at 0 °C, was added dropwise a solution of diethylamine (3.6 g, 50.2 mmol) in ethanol (26 mL). The resulting solution stirred at 0 °C for 1 h, then at room temperature for 3.5 h. The reaction mixture was quenched with ethyl acetate and partitioned with saturated aqueous ammonium chloride solution. The aqueous phase was extracted with ethyl acetate, and the organic extracts were washed with brine. The combined organic extracts were dried over sodium sulfate, filtered, concentrated in vacuo and chromatographed (silica gel, gradient 5 to 10% $CH_2Cl_2$:EtOAc) to yield the title compound as an oil (11.3 g, 87%). $^1H$ NMR (400 MHz, $CDCl_3$) δ 4.25 (q, $J$ = 7.1 Hz, 2 H), 4.02(s, 4 H), 2.92 (t, $J$ = 6.5 Hz, 2 H), 2.74 (s, 2 H), 1.90 (t, $J$ = 6.6Hz, 2H), 1.33 (t, $J$ = 7.1, 3 H).

c) Ethyl 7,7-Ethylenedioxy-4-hydroxy-2-methyl-5,6,7,8-tetrahydrobenzo[b]thieno[2,3-b]pyridine-2-carboxylate

To a solution of compound of Example 3(b) (11.2 g, 39.5 mmol) in toluene (307 mL) at room temperature was added ethyl 3-ethoxycrotonate (12.4 g, 78.6 mmol) and camphorsulfonic acid (0.78 g, 3.4 mmol). The reaction mixture was heated at reflux for 3.5 h using a Dean Stark trap. The mixture was then cooled, and to it was added dropwise a freshly prepared solution of 1 M sodium ethoxide (49 mL). Once the addition was complete the reaction mixture was heated at reflux for 3 h. The mixture was cooled and the precipitate was filtered. The salt was dissolved in methanol (60 mL), to it was added water (500 mL) and acetic acid (2 mL) to yield the title compound as a yellow solid (10.4 g, 76%): mp 94-95 °C; $^1H$ NMR (400 MHz, $CDCl_3$) δ 4.48 (q, $J$ = 7.1 Hz, 2 H), 4.06(s, 4 H), 3.26 (t, $J$ = 6.5 Hz, 2 H), 3.02 (s, 2 H), 2.81 (s, 3 H), 2.02 (t, $J$= 6.5, 2 H), 1.47(t, $J$= 7.1 Hz, 3 H); MS (ESI) $m/z$ 350 $[M + H]^+$; Anal. Calcd. for $C_{17}H_{19}NO_5S$; C, 58.44; H, 5.48; N, 4.01; Found: C, 58.34; H, 5.46; N, 3.86.

d) Ethyl 7,7-Ethylenedioxy4-trifluoromethylsulfonyloxy-2-methyl-5,6,7,8-tetrahydrobenzo[b]thieno[2,3-b]pyridine-3-carboxylate

To a solution of compound of Example 3(c) (5.0 g, 14.3 mmol) in pyridine (50 mL) was added dropwise triflic anhydride (4.0 g, 14.2 mmol). The reaction mixture stirred at 0 °C for 4 h until complete. The reaction mixture was washed with aqueous copper sulfate solution (3x) followed by water (2x), and brine (2x). The organic layer evaporated, dried over anhydrous sodium sulfate and concentrated in vacuo. Purification by flash chromatography (silica gel, 1:1 hexane: ethyl acetate) yielded the title compound as a light yellow solid (3.7 g, 54%): mp 133-134 °C; $^1H$ NMR (400

MHz, $CDCl_3$) δ 4.43 (q, *J* = 7.2 Hz, 2 H), 4.06(s, 4 H), 3.16 (t, *J* = 6.5 Hz, 2 H), 3.10 (s, 2 H), 2.77 (s, 3 H), 2.03 (t, *J* = 6.8 Hz, 2 H), 1.41 (t, *J* = 7.1 Hz, 3 H); MS (ESI) *m/z* 482 $[M + H]^+$; Anal. Calcd. for $C_{18}H_{18}F_3NO_7S_2$; C, 44.90; H, 3.77; N, 2.91; Found: C, 45.03; H, 3.62; N, 2.89.

e) 8,8-Ethylenedioxy-2,3,7,8,9,10-hexahydro-4-methyl-1H-benzo[b]thieno[2,3-b]pyrazolo[3,4-d]pyridin-3-one

To a solution of compound of Example 3(d) (2.4 g, 5.0 mmol) in methanol (40 mL) at room temperature was added hydrazine monohydrate (4.1 g, 82.3 mmol). The reaction mixture was heated at reflux for 3 h. The mixture was cooled then partitioned between pH 7 aqueous buffer and ethyl acetate. The organic layer was dried over anhydrous sodium sulfate, filtered, concentrated in vacuo and recystallized from methanol/ethyl acetate to yield the title compound as a light yellow solid (0.99 g, 60%). $^1H$ NMR (400 MHz, $d_4$-MeOH) δ 4.05 (s, 4 H), 3.15 (t, *J* = 6.5 Hz, 2 H), 3.04 (s, 2 H), 2.82 (s, 3 H), 2.06 (t, *J* = 6.5 Hz, 2 H); MS (ESI) *m/z* 318 $[M + H]^+$; Anal. Calcd. for $C_{15}H_{15}N_3O_3S.0.25\ H_2O$: C, 55.97; H, 4.85; N, 13.05; Found: C, 55.85; H, 4.75; N, 13.30.

Example 4

Preparation of 4-[4-(4-Methylbenzoyl)benzoyl]phenylacetaldehyde (Compound 4)

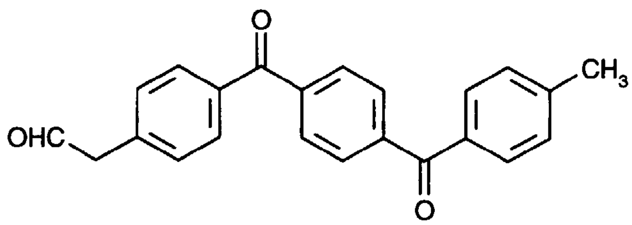

a) Methyl 4-(4-methylbenzoyl)benzoate

A solution of methyl terephthaloyl chloride (6.2 g, 31 mmol) in 250 mL of toluene was treated with aluminum chloride (8.0 g, 60 mmol) at 0°C under an argon atmosphere. The stirring mixture was warmed to 35°C for 0.5 h. and then added slowly to 100 g of ice, followed by 150 mL of ethyl acetate, 50 mL of conc. HCl, and 50 mL of water. The phases were separated, and the aqueous portion was extracted twice with 100 mL of ethyl acetate. The combined organic portionswere washed with water (2 x 75 mL) and brine (1 x 75 mL), dried over magnesium sulfite, filtered, and concentrated to a white solid. Recrystallization from ethyl acetate and hexane afforded 6.0 g (79%) of the title compound as white needles. mp. 117-118°C; $^1H$ NMR (400 MHz, $CDCl_3$) δ 8.15 (d, *J* = 8.35 Hz, 2H), 7.83 (d, *J* = 8.30 Hz, 2H), 7.73 (d, *J* =.8.18 Hz, 2H), 7.31 (d, *J* = 8.04 Hz, 2H), 3.98 (s, 3H), 2.46 (s, 3H); MS (ESI) *m/z* 255 $(M+H)^+$.

b) 4-(4-Methylbenzoyl)benzoic acid

A stirring solution of methyl 4-(4-methylbenzoyl)benzoate (5.00 g, 20.0 mmol in 150 mL of 2:1 ThF : water at 65°C was treated with lithium hydroxide monohydrate (2.0 g, 48 mmol). After a period of 0.5 h the cloudy reaction mixture was allowed to cool to room temperature and treated with ethyl acetate (300 mL) and 10% HCl (aq.). The organic phase was separated, washed with water (2 x 50 mL) and brine (1 x 50 mL), dried over magnesium sulfate, filtered, and concentrated to a white foam. $^1H$ NMR (400 MHz, $CDCl_3$) δ 8.22(d, *J* = 8.34 Hz, 2H), 7.81 (d, *J* = 8.31 Hz, 2H), 7.73 (d, *J* =.8.15 Hz, 2H), 7.31 (d, *J* = 8.00 Hz, 2H), 2.46 (s, 3H).

c) 4-[4-(4-Methylbenzoyl)benzoyl]anisole

A solution of compound of Example 4(b) in 250 mL of toluene was treated with oxalyl chloride (21.8 g, 0.17 mol). The resulting mixture was heated to reflux for 2 h, then concentrated and allowed to stand overnight at 0.5 mm Hg and 25°C. This solid was then dissolved in 100 mL of anisole and treated with aluminum chloride (11.2 g, 84 mmol) at 0°C. The mixture was heated to 70°C for 1 h and then added slowly to 100 g of ice, followed by 150 mL of ethyl acetate, 50 mL of conc. HCl, and 50 mL of water. The phases were separated and the aqueous portion was extracted with 100 mL of ethyl acetate. The combined organic extracts were washed with water (2 x 75 mL) and brine (1 x 75 mL) , dried over magnesium sulfate, filtered, and concentrated to a white solid. Recrystallization from ethyl acetate and hexane yielded 4.6 g (70%) of the title compound. mp. 167-169°C; $^1H$ NMR (400 MHz, $CDCl_3$) δ .7.8-7.9 (m, 6H), 7.77 (d, *J* = 8.06 Hz, 2H), 7.32 (d, *J* = 8.01 Hz, 2H), 7.0 (d, *J* = 8.74 Hz, 2H), 3.92 (s, 3H), 2.47 (s, 3H); MS (ESI) *m/z* 331 $(M+H)^+$.

d) 4-[4-(4-Methylbenzoyl)benzoyl]phenol

A solution of compound of Example 4(c) (700 mg, 2.12 mmol) in 20 mL of dichloromethane was treated with aluminum chloride (1.0 g, 7.5 mmol) and 7.0 mL of 1.0 M boron trichloride solution in dichloromethane and heated to reflux for 1 h. The mixture was then diluted with 100 mL of dichloromethane and washed with 10% HCl (aq) (1 x 25 mL), water (1 x 25 mL), and brine (1 x 25 mL). The organic phase was dried over magnesium sulfate, filtered, and concentrated to a dark residue which was subjected to flash chromatography (silica gel, elution with 1:1 ethyl acetate : hexane) to yield 550 mg (82%) of the title compound. $^1$H NMR (400 MHz, $CDCl_3$) δ .7.8-7.9 (m, 6H), 7.77 (d, *J* = 8.05 Hz, 2H), 7.32 (d, *J* = 8.01 Hz, 2H), 6.93 (d, *J* = 8.6 Hz, 2H), 2.47 (s, 3H).

e) 4-[4-(4-Methylbenzoyl)benzoyl]phenyl trifluoromethylsulfonate

A solution of compound of Example 4(d) (320 mg, 1.0 mmol) in THE (20 mL) was treated with sodium hydride (40 mg, 1.67 mmol) and N-phenyltrifluoromethanesulfonimide (500 mg, 1.40 mmol) at 0 °C. The reaction mixture was allowed to warm up to room temperature and was then stirred for 18 h. room temperature. The reaction was then partitioned between ethyl acetate and brine; layers were separated and the organic extract was dried over magnesium sulfate and evaporated. Purification by flash chromatography (silica gel, 80:20 hexane : ethyl acetate) afforded the title compound (300 mg, 66%). mp. 180-181°C; $^1$H NMR (400 MHz, $CDCl_3$) δ 7.96 (d, *J* = 8.6 Hz, 2 H), 7.89 (s, 4 H), 7.76 (d, *J* = 8.1 Hz, 2 H), 7.45 (d, *J* = 8.6 Hz, 2 H), 7.33 (d, *J* = 8.1 Hz, 2 H), 2.47 (s, 3 H).

f) 4-[4-(4-Methylbenzoyl)benzoyl]phenylacetaldehyde

To a solution of compound of Example 4(e) (445 mg, 1.0 mmol) in DMF (10 mL) was added allyltributyltin (0.35 mL, 1.12 mmol), bis(triphenylphosphine)palladium(II) chloride (55 mg, 0.077 mmol) and lithium chloride (125 mg, 2.95 mmol). The reaction mixture was heated to 90 °C for 1 h, and then allowed to cool to room temperature before being partitioned between ethyl acetate and brine. The organic layer was dried over magnesium sulfate and concentrated to a residue consisting of the desired product, 3-[4-[4-(4-methylbenzoyl)benzoyl]phenyl]-1-propene, and tin-containing by-products. This material was subjected to flash chromatography (silica gel, elution with 95:5 hexane : ethyl acetate) which removed most, but not all, of the tin impurities. A second chromatography (gradient 5% to 10% ethyl acetate in hexane) yielded 100 mg (30%) of clean olefin, which was then dissolved in dichloromethane/methanol (3:1, 16 mL) at -78 °C. Ozone was bubbled through this solution for 5 min. The reaction was quenched with five drops of dimethyl sulfide and stirring continued for 30 min at -78 °C. The solvent was evaporated and the resulting material purified by flash chromatography (silica gel, elution with gradient 85:15 to 75:25 hexane : ethyl acetate) to yield the title compound (40 mg, 40%). mp. 188-190°C; $^1$H NMR (400 MHz, $CDCl_3$) δ 9.83 (s, 1 H), 7.88 (s, 4 H), 7.86 (d, *J* = 8.1 Hz, 2 H), 4.03 (s, 4 H), 3.73 (s, 3 H), 3.76 (t, J = 6.0 Hz, 2 H), 3.00 (s, 2 H), 2.80 (s, 3 H), 2.03 (t, J = 6.0 Hz, 2 H); MS (ESI) *m/z* 343 $(M+H)^+$.

Example 5

Preparation of 1,4-Dimethyl-8,8-ethylenedioxy-2,3,7,8,9,10-hexahydro-1H-benzo[b]thieno[2,3-b]pyrazolo[3,4-d] pyridin-3-one (Compound 5)

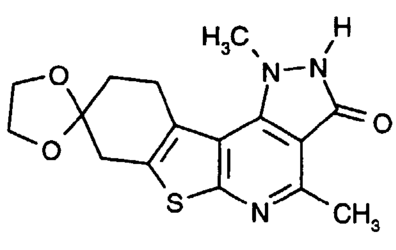

1,4-Dimethyl-8,8-ethylenedioxy-2,3,7,8,9,10-hexahydro-1H-benzo[b]thieno[2,3-b]pyrazolo[3,4-d]pyridin-3-one

A solution of compound of Example 3(d) (0.4 g, 0.83 mmol) in methanol (6.7 mL) at room temperature was treated with methylhydrazine (0.16 g, 3.45 mmol) and the mixture is heated at reflux for 2 h. The mixture was cooled and the precipitate, containing 150 mg of the 2,4-dimethyl regioisomer, filtered. The filtrate was evaporatewd and purified by flash chromatography (silica gel, elution with 80:20:5 ethyl acetate:methanol:acetic acid) to provide the title compound as a yellow solid (22 mg). $^1$H NMR (400 MHz, $CDCl_3$) δ 4.03 (s, 4 H), 3.73 (s, 3 H), 3.76 (t, *J* = 6.0 Hz, 2 H), 3.00 (s,

2 H), 2.80 (s, 3 H), 2.03 (t, *J* = 6.0 Hz, 2 H); MS (ESI) *m/z* 332 $(M+H)^+$.

Example 6

Preparation of 4-carboxy-benzophenone-4-carboxamido-trans-4-methyl-cyclohexyl-N-hexyl carboxamide (Compound 6)

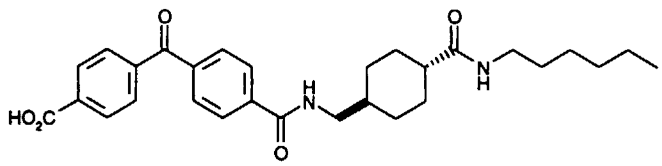

a) N-t-butyloxy carbonyl-trans-4-aminomethyl cyclohexyl carboxylic acid

Aqueous sodium hydroxide (1N, 100 ml, 100 mmol) was added to a solution of 4-trans-aminomethyl-cyclohexyl-carboxylic acid (9.0g, 60 mmol), in dioxane (100 ml), water (100 ml) at 0 degrees C. Boc anhydride (15.9 g, 66 mmol) was added and the reaction was warmed to rt and stirred overnight. The solution was concentrated to 50 ml, then was diluted with EtOAc (100 ml) and acidified to pH 2 with adqueous $KHSO_4$ (1N). The organic layer was then extracted with water (100 ml) two times, and the organics were concentrated in vacuo. The solid was recrystallized from EtOAc/hexanes to yield 9.2g + 3.4g (second crop) of a white solid. (80% yield). MS (ES) m/e 242 $[M+H]^+$.

b) N-t-butyloxy carbonyl-trans-4-aminomethyl cyclohexyl (Kaiser oxime resin) carboxylate

Kaiser oxime resin (20 g, 0.7 mmol/g loading, Advanced Chem Tech) was added to a solution of N-t-butyloxy carbonyl-trans-4-aminomethyl cyclohexyl carboxylic acid (5.0 g, 20 mmol) and DCC (4.4 g, 20 mmol) in methylene chloride (200 ml) and was gentle mixed at rt overnight. The solid was filtered and collected, then washed with methylene chloride (5 x 100 ml). Then the resin was resuspended in methylene chloride (200 ml), and N-t-butyloxy carbonyl-trans-4-aminomethyl cyclohexyl carboxylic acid (5.0 g, 20 mmol) and DCC (4.4 g, 20 mmol) were added and the reaction was gently mixed overnight at rt. The solid was filtered and collected, then washed with methylene chloride (5 x 100 ml), then was dried overnight under vacuum. IR (KBr, $cm^{-1}$)=1820, 1771, 1520.

c) trans-4-aminomethyl cyclohexyl (Kaiser oxime resin) carboxylate

N-t-butyloxy carbonyl-trans-4-aminomethyl cyclohexyl (Kaiser oxime resin) carboxylate (20 g) was suspended in methylene chloride (100 ml) and TFA (25 ml) was added. The reaction was gently mixed for 0.5 h, then the solid was filtered and collected, then was washed with with methylene chloride (5 x 100 ml), then was dried overnight under vacuum. IR (KBr, $cm^{-1}$)=3150, 1770, 1526..

d) 4-carboxy-benzophenone-4-carboxamido-trans-4-methyl-cyclohexyl-(Kaiser oxime resin) carboxylate

Trans-4-aminomethyl cyclohexyl (Kaiser oxime resin) carboxylate (200 mg) was suspended in DMF (3.0 ml) and N-methyl morpholine (0.2 ml) and 4, 4'-benzophenone dicarboxylic acid (190 mg, 0.7 mmol) and HBTU (265 mg, 0.7 mmol) was added and the reaction was gently mixed for 3 h. The solid was filtered and collected, then was washed with with DMF (3 x 20 ml), then water (3 x 20 ml), then was resuspended in DMF (3.0 ml) and N-methyl morpholine (0.1 ml) and 4,4'-benzophenone dicarboxylic acid (0.35 mmol) and HBTU (0.35 mmol) was added and the reaction was gently mixed for 3 h. The solid was filtered and collected, then was washed with with DMF (3 x 20 ml), then water (3 x 20 ml), then methylene chloride (5 x 20 ml), then was dried under vacuum.

e) 4-carboxy-benzophenone-4-carboxamido-trans-4-methyl-cyclohexyl-N-hexyl carboxamide

4-carboxy-benzophenone-4-carboxamido-trans-4-methyl-cyclohexyl-(Kaiser oxime resin) carboxylate (200 mg) was suspended in methylene chloride (3.0 ml) and hexyl amine (0.3 mmol) was added. The reaction was gently mixed for 3 h then was filtered, and the filtrate was concentrated in vacuo to yield the title compound: MS (ES) m/e 493 $[M+H]^+$.

Example 7

Preparation of 4-nitro-benzamido-trans-4-methyl-cyclohexyl-N-hexyl carboxamide (Compound 7)

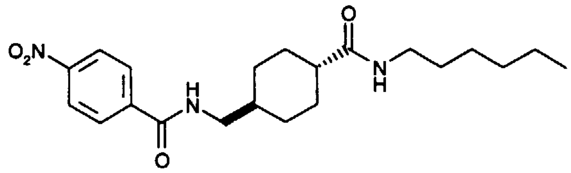

4-nitro-benzamido-trans-4-methyl-cyclohexyl-N-hexyl carboxamide

Following the procedure of Example 6(a)-(e), except substituting 4-nitro benzoic acid for and 4, 4'-benzophenone dicarboxylic acid, the title compound was prepared: MS (ES) m/e 390 $[M+H]^+$.

Example 8

Preparation of 4-acetamido-benzamido-trans-4-methyl-cyclohexyl-N-1-(amino-R-2-(methoxy methyl)-pyrrolidine) carboxamide (Compound 8)

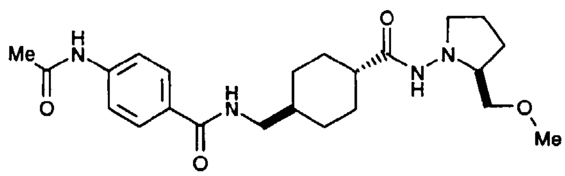

Following the procedure of Example 6(a)-(e), except substituting 4-acetamido- benzoic acid for and 4, 4'-benzophenone dicarboxylic acid and R-1-amino-2-(methoxy methyl)- pyrrolidine (RAMP) for hexyl amine, the title compound was prepared: MS (ES) m/e 331 $[M+H]^+$.

Example 9

Preparation of 4-formyl-E-cinnamido-trans-4-methyl-cyclohexyl-N-(propyl) carboxamide (Compound 9)

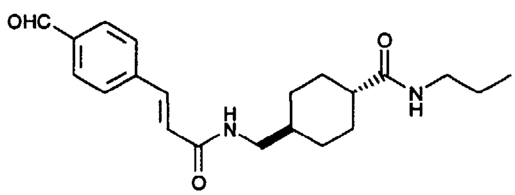

Following the procedure of Example 6(a)-(e), except substituting 4-formyl cinnamic acid for and 4, 4'-benzophenone dicarboxylic acid and propyl amine for hexyl amine, the title compound was prepared: MS (ES) m/e 357 $[M+H]^+$.

Example 10

Preparation of 2,3,7,8,9,10-Hexahydro-4-methyl-1H-benzo[b]thieno[2,3-b]pyrazolo[3,4-d]pyridin-3-one (Compound 10)

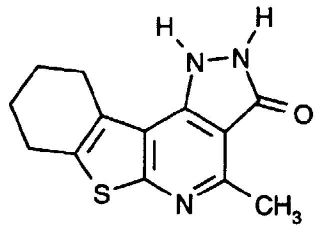

a) Ethyl 4-Hydroxy-2-methyl-5,6,7,8-tetrahydrobenzo[b]thieno[2,3-b]pyridine-2-carboxylate

A solution of ethyl 2-amino-4,5,6,7-tetrahydrobenzo[b]thiophene-3-carboxylate (8.9 g, 39 mmol) and ethyl 3-ethoxycrotonate (12.4 g, 78 mmol) in toluene (300 mL) was treated with camphorsulfonic acid (0.78 g, 3.4 mmol) and the reaction mixture was heated at reflux for 3 h using a Dean Stark trap. The mixture was then cooled to room temperature and was subsequently treatedwith a freshly prepared 1 M solution of sodium ethoxide (48 mL, 48 mmol). After addition was complete the reaction mixture was heated at reflux for 3 h. The mixture was cooled, concentrated and the residue dissolved in ethyl acetate. Acetic acid (2 mL) was added, solvent evaporated and resulting solid triturated with methanol to yield the title compound as an off-white solid (8.4 g, 74%): mp 140 °C; $^1H$ NMR (400 MHz, $CDCl_3$) δ 4.48 (q, $J$ = 7.2 Hz, 2 H), 3.04 (br s, 2 H), 2.81 (s, 3 H), 2.80 (br s, 2 H), 1.87 (br s, 4 H), 1.47 (t, $J$ = 7.2 Hz, 3 H);; Anal. Calcd. for $C_{15}H_{17}NO_3S$: C, 61.83; H, 5.88; N, 4.81; Found: C, 61.69; H, 5.81; N, 4.73.

b) Ethyl 4-Chloro-2-methyl-5,6,7,8-tetrahydrobenzo[b]thieno[2,3-b]pyridine-2-carboxylate

A solution of compound of Example 10(a) (8.0 g, 27.4 mmol) in phosphorus oxychloride (100 mL) was refluxed for 3.5 hours. The phosphorus oxychloride was removed under vacuum and the residual oil was dissolved in ethyl acetate, washed with 5% aqueous sodium bicarbonate and dried over anhydrous sodium sulfate. Evaporation of the solvent provided the title compound as a crystalline solid (8.5 g, 95%): mp 65-66 °C; $^1H$ NMR (400 MHz, $CDCl_3$) δ 4.47 (q, $J$ = 7.1 Hz, 2 H), 3.10 (br s, 2 H), 2.85 (br s, 2 H), 2.60 (s, 3 H), 1.89 (br s, 4 H), 1.43 (t, $J$ = 7.1 Hz, 3 H); Anal. Calcd. for $C_{15}H_{16}ClNO_2S.0.125\ H_2O$: C, 57.73; H, 5.25; N, 4.49; Found: C, 57.69; H, 5.08; N, 4.30.

c) 2,3,7,8,9,10-Hexahydro-4-methyl-1H-benzo[b]thieno[2,3-b]pyrazolo[3,4-d]pyridin-3-one

A solution of compound of Example 10(b) (2.0 g, 6.4 mmol) in methanol (50 mL) was treated with hydrazine monohydrate (10 mL) and the resulting mixture was heated at reflux for 16 h. The reaction was poured over diluted aqueous hydrochloric acid and the title compound precipitated as a yellow solid (1.8 g). $^1H$ NMR (400 MHz, $d_4$-MeOH) δ 3.01 (br s, 2 H), 3.00 (s, 3 H), 2.92 (br s, 2 H), 2.00 (br s, 4 H); Anal. Calcd. for $C_{13}H_{13}N_3OS.HCl.0.25\ H_2O$: C, 52.00; H, 4.87; N, 13.99; Found: C, 51.92; H, 5.01; N, 13.70.

Example 11-Protocol for the Determination of the Potency of SH2 Domain Antagonists

The inhibitory activity of compounds at the different human SH2 domains was determined in vitro using SH2 domains expressed as fusion proteins in E. coli. The SH2 domains used herein were the human forms of the src SH2 domain, Grb2 SH2 domain, lck SH2 domain, fyn SH2 domain, SH-PTP2 SH2 domain, p85 SH2 domain and hcp SH2 domain.

The fusion proteins containing the src, lck and hcp SH2 domains were expressed as the general sequence: DET1-DET2-spacer-ek-SH2, where DETI, DET2, spacer, ek and SH2 are as described below. DET1 ("defined epitope tag 1") (SEQ ID NO: 1) is an 11 amino acid sequence found in the Human Immunodeficiency Virus Type 1 (HIV-1) envelope protein gp120 (or gp160). Monoclonal antibodies to various epitopes of HIV-1 gp120 (or gp160) are known in the art, see, for example U.S. Patent 5,166,050. One preferred example is monoclonal antibody 178.1 (see, e.g., Thiriart et al., J. Immunol., 143:1832-1836 (1989)), which was prepared by immunization of mice with a yeast-expressed HIV-1 gp160 molecule from strain BH10 (Ratner et al., Nature, 313:277-284 (1985)). This tag was used for detection of expression (by Western blot), for purification of the protein (by affinity chromatography), and for configuring assays in which the fusion protein was captured or immobilized using the 178.1 antibody. DET2 is a hexahistidine sequence

tag (SEQ ID NO: 2) which binds to nickel-containing resins and was used for purification purposes. Spacer (SEQ ID NO: 3) was utilized to design a BamH1 restriction site at the indicated position of the construct. The term -ekrefers to a recognition sequence (SEQ ID NO: 4) for the enterokinase protease which provides for the optional removal of the tags from the SH2 domain, thus producing an SH2 domain that contains no extraneous amino acids. SH2 domains which contain no extraneous amino acids are preferable to tagged protein for crystallography studies. SH2 refers to the SH2 domains of different proteins.

The DNA sequence encoding each DET1-DET2-spacer-ek-SH2 was designed such that the indicated restriction sites (BamH1 and XbaI) flank the spacer-ek-SH2 region, thereby allowing different spacer-ek-SH2 contructs to be readily substituted into any one of the vectors described in Procedures 2 or 3 below to create a DET1-DET2-spacer-ek-SH2 tagged protein. The DNA sequence encoding each DET1-DET2-spacer-ek-SH2 constructs was also designed such that the entire tagged SH2 domain can be moved as an NdeI-XbaI fragment into any expression vector containing an NdeI site at an appropriate distance downstream of E. coli transcription and translation regulatory sequences and a downstream cloning site compatible with XbaI. Although any suitable vector would yield similar results(e.g., pET-11a; Novagen, Inc.), the vector used in the instant experiments was E. coli expression vector pEA1KnRBS3. This vector is a derivative of the series of vectors described in Shatzman, A, Gross, M, and Rosenberg, M, 1990, "Expression using vectors with phage lambda regulatory sequences", In: Current Protocols in Molecular Biology (F.A. Ausubel et al , eds.), pp. 16.3.1-16.3.11, Greene Publishing and Wiley-Interscience, N.Y. (hereinafter F.A. Ausubel et al.). The specific vector pEA1KnRBS3 is described in Bergsma et al, 1991, J. Biol. Chem. 266:23204-23214.

The procedures below describe the expression of chicken src, human src, human lck and human hcp SH2 domains. First, the chicken src SH2 domain was expressed as DET1-DET2-spacer-SH2. Then, the others were inserted into this vector in place of chicken src to express proteins in the form DET1-DET2-spacer-ek-spacer-SH2.

Procedure 1:

Cloning and Expression of chicken src SH2 domain containing tags DET1 and DET2 (DET1-DET2-spacer-SH2).

A DNA sequence encoding the tagged protein DET1-DET2-spacer-SH2 was PCR amplified from a cDNA clone containing the chicken src gene (p5H; Levy et al 1986. Proc. Natl. Acad. Sci. USA 83:4228) by methods well known to those skilled in the art by using the following primers:

5'

TTCCATATGAAAAGTATTCGTATTCAGCGTGGCCCGGGCCGTCACCACCA

CCACCACCACGGGATCCCCGCTGAAGAGTGGTACTTT 3' (SEQ ID NO: 17)

The underlined sites are an NdeI recognition site (5') and a BamHI recognition site (3').

5' GGAATTCTAGATTACTAGGACGTGGGGCAGACGTT 3' (SEQ ID NO: 18)

The underlined region is an XbaI recognition site.

The PCR product was digested with NdeI and XbaI, followed by isolation of the digested fragment on an agarose gel. The fragment was ligated into NdeI-XbaI-digested pEAKnRBS3 vector (Bergsma et al, supra) that had been agarose gel purified as a 6.5 kbp fragment. The ligation reaction was used to transform E. coli MM294cI$^+$ (F.A. Ausubel et al., supra). A plasmid containing an insertion of the correct fragment was identified and confirmed by DNA sequencing. The resultant plasmid encodes DET1-DET2-spacer-SH2 under the control of the phage lamda $P_L$ promoter and regulatory system. Plasmid DNA was purified from MM294cI$^+$ and used to transform E. coli strain AR120. In this host strain, expression of the phage promoter can be induced by addition of nalidixic acid to the growing culture as described in F.A. Ausubel et al, supra. Nalidixic acid induction of AR120 containing this plasmid, followed by analysis of the cellular proteins on an SDS-polyacrylamide gel stained with Coomassie Blue (F.A. Ausubel et al., supra), resulted in appearance of a protein band with an apparent molecular weight of 15,000; this band was not seen in uninduced cells or in induced cells containing pEA1KnRBS3 lacking the PCR amplified fragment. Western blotting confirmed that the induced protein band reacted with the anti-DET1 monoclonal antibody 178.1.

Procedure 2:

Cloning, expression and purification of human src SH2 domain containing tags and an enterokinase proteolytic cleavage site (DET1-DET2-spacer-ek-src SH2).

A DNA sequence encoding protein ek-src SH2 was PCR amplified from a cDNA clone containing the human src gene (c-src SH2 DNA sequence identical to that described in Takeya,T. and Hanafusa, H, 1983 Cell 32:881-890) using the following primers:

5' CGGGATCCTGGACGACGACGACAAAGCTGAGGAGTGGTATTTT 3' (SEQ ID NO: 19)

The underlined site is a BamHI recognition site.

5' GGAATTCTAGACTATTAGGACGTGGGGCACACGGT 3' (SEQ ID NO: 20)

The underlined region is an XbaI recognition site.

The PCR product was digested with BamHI and XbaI, followed by isolation of the digested fragment on an agarose gel. The fragment was ligated into BamHI-XbaI-digested expression vector containing the tagged chicken src gene DET1-DET2-spacer-SH2 described in Procedure 1 above. In that vector, the BamHI site is located between the coding regions for DET2 and SH2, and the XbaI site is located after the 3' end of the SH2 coding region. The ligation reaction was used to transform E. coli MM294cI+. The construct DET1-DET2-spacer-ek-src SH2 was confirmed by DNA sequencing (SEQ ID NO: 5) and induced in E. coli strain AR120 as described in Procedure 1 above. A Coomassie-Blue-stained, Western-blot-positive induced protein band with an apparent molecular weight of 16,000 was observed after nalidixic acid induction.

Cells were lysed at neutral pH by sonication in the presance of lysozyme. After centrifugation, the soluble extract was chromatographed on a Ni++NTA column. After washing the column with equilibration buffer (Tris buffer pH 8 containing 0.5 M NaCl) and the same buffer containing 15 mM imidazole, the protein was eluted in highly purified form with 25 mM imidazole in equilibration buffer. The SH2 domain, purified in this fashion, was found to bind with high affinity in a specific, saturable fashion to the appropriate pY peptide in the "Binding Assays" described below, demonstrating that the tag did not interfere with function. This expressed fusion protein, DET-DET2-spacer-ek-src SH2, was utilized in the "Binding Assays" described below in order to determine the specificity of compounds to selectively inhibit the human src SH2 domain.

Procedure 3:

Cloning and expression of human lck SH2 domain containing tags and an enterokinase proteolytic cleavage site (DET1-DET2-spacer-ek-lck SH2).

A DNA sequence encoding protein ek-lck SH2 was PCR amplified from a cDNA clone containing the human lck gene (Genbank accession number M36881) using the following primers:

5' CGGGATCCTGGACGACGACGACAAAGAGCCCGAACCCTGGTTCTT 3' (SEQ ID NO: 21)

The underlined site is a BamHI recognition site.

5' GCTCTAGACTATTACTGGGGCTTCTGGGTCTG 3' (SEQ ID NO: 22)

The underlined region is an XbaI recognition site.

The PCR product was digested with BamHI and XbaI, followed by isolation of the digested fragment on an agarose gel. The fragment was ligated into BamHI-XbaI-digested expression vector containing the tagged chicken src gene DET1-DET2-spacer-SH2 described in Procedure 1 above. In that vector, the BamHI site is located in between the coding regions for DET2 and SH2, and the XbaI site is located after the 3' end of the SH2 coding region. Thus, the ek-lck SH2 sequence replaced the src SH2 sequence in the above vector. The ligation reaction was used to transform E. coli MM294cI+. The construct containing DET1-DET1-spacer-ek-lck SH2 was confirmed by DNA sequencing (SEQ ID NO: 6) and induced in E. coli strain AR120 as described in Procedure 1 above. A Coomassie-Blue-stained, Western-blot-positive induced protein band with an apparent molecular weight of 17,000 was observed after nalidixic acid induction.

Cells were lysed at neutral pH by sonication in the presance of lysozyme. After centrifugation, the soluble extract was chromatographed on a Ni++NTA column. After washing the column with equilibration buffer (Tris buffer pH 8 containing 0.5 M NaCl) and the same buffer containing 15 mM imidazole, the protein was eluted in highly purified form

with 25 mM imidazole in equilibration buffer. The SH2 domain, purified in this fashion, was found to bind with high affinity in a specific, saturable fashion to the appropriate pY peptide in the "Binding Assays" described below, demonstrating that the tag did not interfere with function. This expressed fusion protein, DET1-DET2-spacer-ek-lck SH2, was utilized in the "Binding Assays" described below in order to determine the specificity of compounds to selectively inhibit the human lck SH2 domain.

Procedure 4:

Cloning and expression of human hcp SH2 domain containing tags and an enterokinase proteolytic cleavage site (DET1-DET2-spacer-ek-hcp SH2).

A DNA sequence encoding protein ek-hcp SH2 was reverse transcriptase-PCR amplified from human fetal liver RNA. RNA isolation used Tri-Reagent (Molecular Research Center Inc.) and the Reverse Transcriptase system (GIBCO-BRL) according to the manufacture's instructions. PCR was carried out using the following primers:

5' GAAGATCTTGGACGACGACGACAAATCCCGTGGGTGGTTTCAC 3'(SEQ ID NO: 23)

The underlined site is a BglII recognition site.

5' GCTCTAGACTATTAACTAGTGGGATCGGAGCA 3' (SEQ ID NO: 24)

The underlined region is an XbaI recognition site.

The PCR product was digested with BglII and XbaI, followed by isolation of the digested fragment on an agarose gel. The fragment was ligated into BamHI-XbaI-digested expression vector containing the tagged human src gene DET1-DET2-spacer-ek-src SH2 described in Procedure 2 above. In that vector, the BamHI site is located in between the coding regions for DET2 and ek, and the XbaI site is located after the 3' end of the SH2 coding region. Thus, the ek-hcp SH2 sequence replaced the ek-src SH2 sequence in the above vector. The ligation reaction was used to transform E. coli MM294cI$^{+}$. The construct containing DET1-DET2-spacer-ek-hcp SH2 was confirmed by DNA sequencing (SEQ ID NO: 7) and used to transform E. coli GI698 (Invitrogen Corporation, San Diego, CA). Induction of the phage lambda promoter was induced by addition of tryptophan to the culture medium to 10 mg/ml, per the manufacture's instructions. A Coomassie-Blue-stained, Western-blot-positive induced protein band with an apparent molecular weight of 15,000 was observed after tryptophan induction of cells growing at 30° C.

Cells were lysed at neutral pH by sonication in the presance of lysozyme. After centrifugation, the insoluble pellet was solubilized with 8 M urea in Tris buffer pH 8 and bound onto a Ni$^{++}$NTA column. The resin was washed with equilibration buffer (Tris buffer pH 8 containing 0.5 M NaCl, 8 M urea and 5 mM BME) and the same buffer containing 15 mM imidazole. The protein was refolded on the column during the removal of urea in the presence of 5 mM BME and the purified refolded protein eluted with 300 mM imidazole in Tris buffer pH 8. The SH2 domain, purified in this fashion, was found to bind with high affinity in a specific, saturable fashion to the appropriate pY peptide in the "Binding Assays" described below, demonstrating that the tag did not interfere with function and that the protein was refolded successfully. This expressed fusion protein, DET1-DET2-spacer-ek-hcp SH2, was utilized in the "Binding Assays" described below in order to determine the specificity of compounds to selectively inhibit the human hcp SH2 domain.

Fusion proteins having the structure GST-X-SH2 were prepared as described in the GST gene fusion kit system available from Pharmacia (New Jersey). GST is the tagging sequence glutathione s-transferase epitope (SEQ ID NO: 8) for fyn, Grb2 and SH-PTP2 and is the tagging sequence glutathione s-transferase epitope (SEQ ID NO: 9) for p85. SH2 refers to the SH2 domains of fyn, Grb2, p85 and SH-PTP2 which were expressed and purified using glutathione Sepharose 4B (Pharmacia) according to "Current Protocols in Molecular Biology", ed. FM Ausubel et al., pub. John Wiley and Sons, Inc., (1995), p 16.7.1. X is an appropriate linker, preferably of 6 to 21 base pairs, used to keep the SH2 construct in frame and complement cloning. As such, the sequence of X is not critical. One skilled in the art can readily construct the appropriate linker. The DNA sequence encoding each GST-X-SH2 fusion protein was designed such that the indicated restriction sites (BamH1 and EcoRI) flank the SH2 region. The vector used in the instant experiments was the E. coli expression vector pGEX-2T (Pharmacia) for fyn, Grb2 and SH-PTP2, and pGEX-3X (Pharmacia) for p85. Each of these vectors result in SH2 constructs having additional C-terminal amino acids as described below.

The sequence encoding the SH2 domain of human fyn (amino acids 143-252) (Yamamoto, T. et al. Proc. Natl. Acad. Sci. USA 83, 5459-5463 (1986)) was cloned into the BamHI and EcoRI sites of the expression vector pGEX-2T. The SH2 domain including the additional C-terminal amino acids leucine-threonine-asparagine-serine-serine (SEQ ID NO: 10) was cloned by PCR techniques known to those skilled in the art to yield the expressed fusion protein GST-X-fyn. This expressed fusion protein was then utilized in the "Binding Assays" described below in order to determine the specificity of compounds to selectively inhibit the human fyn SH2 domain.

Human p85 SH2 domain: The sequence encoding the SH2 domain of human p85 (amino acids 321-440) (Skolnik, E. et al., Cell 65, 83-90 (1991)) was cloned into the BamHI and EcoRI sites of the expression vector pGEX-3X. The SH2 domain including the additional C-terminal amino acids asparagine-serine-serine (SEQ ID NO: 11)was cloned by PCR techniques known to those skilled in the art to yield the expressed fusion protein GST-X-p85. This expressed fusion protein was then utilized in the "Binding Assays" described below in order to determine the specificity of compounds to selectively inhibit the human p85 SH2 domain.

Human SH-PTP2 SH2 domain: The sequence encoding the SH2 domain of human SH-PTP2 (amino acids 1-106)) (Bastien, L. et al., Biochem. Biophys. Res. Commun. 196, 124-133 (1993)) was cloned into the BamHI and EcoRI sites of the expression vector pGEX-2T. The SH2 domain including the additional C-terminal amino acids glutamine-phenylalanine-isoleucine-valine-threonine-aspartate (SEQ ID NO: 12) was cloned by PCR techniques known to those skilled in the art to yield the expressed fusion protein GST-X-SH-PTP2. This expressed fusion protein was then utilized in the "Binding Assays" described below in order to determine the specificity of compounds to selectively inhibit the human SH-PTP2 SH2 domain.

Human Grb2 SH2 domain: The sequence encoding the SH2 domain of human Grb2 (amino acids 58-159) (Lowenstein, E. et al., Cell 70, 431-442 (1992)) was cloned into the BamHI and EcoRI sites of the expression vector pGEX-2T. The SH2 domain including the additional C-terminal amino acids isoleucine-histidine-arginine-aspartate (SEQ ID NO: 25) was cloned by PCR techniques known to those skilled in the art to yield the expressed fusion protein GST-X-Grb2. A six nucleotide linker was used and resulted in the amino acids glycine and serine between the GST and SH2 domain. This expressed fusion protein was then utilized in the "Binding Assays" described below in order to determine the specificity of compounds to selectively inhibit the human Grb2 SH2 domain.

Binding Assays: The potency of compounds at the SH2 domains was determined based on the ability of such compound to selectively inhibit such SH2 domain from binding to its respective specific pY peptide.

The binding assays for the SH2 domains and pY peptides were performed in an ELISA-based 96 well plate assay. In Millipore 96 well filter plates, hydrophilic Durapore® (pore size 0.65um Cat. No. MADVN6550), was added 2 ul (50% suspension) of Protein-G Sepharose (available from Pharmacia of N.J. Cat. No. 17-0618-01) and either 2 ul of 2 mg/ml of MAB178. (for gp120/SH2 domain fusion proteins src, lck and hcp) or 0.25 ul of anti-GST polyclonal antisera (available from Pharmacia of N.J.) (for GST/SH2 domain fusion proteins fyn, Grb2, p85 and SH-PTP2). 10 pmol of the subject SH2 domain fusion protein were added to their respective wells. The volume was brought to 100 ul with TBS-T (tris buffered saline plus 0.05% tween-20), incubated and shaken at room trmperature for 1 hr. then washed 1x with TBS-T (4°C). 90 ul of TBS-T were then added to each well. Specific pY biotinylated peptides were diluted to a concentration of 1.0 uM in TBS-T (these peptides can be obtained from Bachem Bioscience of Pennsylvania, Genosys Biotechnologies of Texas and California Peptide Research of California). 10 ul was aliquoted per well to yield a final concentration of 0.1 uM (approx, the $K_d$ for each SH2 domain/peptide pair) and a final volume of 100 ul. The assay plates were incubated until equilibrium binding was attained (3 hr at 4°C with shaking). The assay plates were washed 2 X per well TBS-T (4°C), then 100 ul of SABC (Strepavidin biotinylated horseradish peroxidase complex, available from the Zymed corporation of California cat. no. 93-0043, 1 drop reagent A (streptavidin) and 1 drop of reagent B (AH-biotin conjugated-horseradish peroxidase) per 10 ml of TBS-T, incubated at 37°C for 30 minutes, then cooled to 4°C) was added per well, then incubated at 4°C for 30-60 minutes. The plates were then washed 4 X with TBS-T (4°C) (250 ul/well)/wash). 100 ul of 1 mg/ml OPD (o-phenyldiamine, Sigma Chemical Corporation, St. Louis Missouri) in Citrate Buffer was added per well. To stop development, 100 ul of 10% sulfuric acid was added per well. 150 ul from each well was then removed from the assay plate and placed in an ELISA plate. The $A_{490}$ of each ELISA plate was then determined.

Determination of ($IC_{50}$) for Table I

Each control or compound was assayed in duplicate. The duplicates were averaged and the background subtracted and the maximal values with no inhibition were taken from the plate, then all other data points were expressed as a percent of the maximal value (or as % control). These % control data values were graphed in Kaleidagraph for Macintosh (Synergy Software). The curves on these graphs were nonlinear curve fitted with the following equation F(x)=Emax/(1+($k_d$/conc)^slope), wherein the $k_d$ term represents the $IC_{50}$ for each of the curves.

Determination of (Ki) for Table II

The Ki for respective compounds is calculated via the following equation (see reference). This expanded equation

must be used under the conditions of this assay, due to the fact that the pY biotinylated peptide is not in vast excess concentration (100X) over the SH2 domain fusion protein. The $IC_{50}$ is an extrapolated value from a nonlinear curve fit using Kaleidagraph. Rtot and *D are known values for reagents input into the assay. KD generally must be experimentally determined for each combination of SH2 domain fusion protein and pY biotinylated peptide.

$$KI=(IC_{50}-Rtot+Rtot/2((*D/(KD+*D))+(KD/(KD+*D+Rtot/2)))/(1+*D/KD+Rtot/KD((KD+*D\ /2)/(KD+*D)))$$

KI=(uM)KDof competitor
$IC_{50}$=(uM) $IC_{50}$ for inhibitor, derived via nonlinear curve fit of competition selectivity assay data for each SH2 domain
Rtot=(uM)total SH2 domain concentration within 1 assay (microtitre plate) well
*D=(uM)concentration of specific pY and biotinylated peptide for each SH2 domain
KD=(uM)KD value for the specific pY and biotinylated peptide for each SH2 domain
$IC_{50}$ is the concentration of inhibitor at which the response or signal is inhibited by 50%
KD is the dissociation constant for a ligand in a receptor/ligand interaction, normally equaling the concentration of ligand which is at 1/2 Vmax on a saturation binding curve>

The pY peptide ligands used in the above Binding Assays are as follows:

Biotinylated pY peptide ligand containing an aminocaproic acid (Aca) linker used for src, lck, and fyn SH2 domains

Glu-Pro-Gln-pTyr-Glu-Glu-Ile-Pro-Ile-Tyr-Leu (SEQ ID NO: 13)

Biotinylated pY peptide ligand containing an aminocaproic acid (Aca) linker used for p85 SH2

Asp-Gly-Gly-pTyr-Met-Asp-Met-Ser-Lys-Asp-Glu (SEQ ID NO: 14)

Biotinylated pY peptide ligand containing an aminocaproic acid (Aca) linker used for SH-PTP2 SH2

Glu-Asn-Gly-Leu-Asn-pTyr-Ile-Asp-Leu-Asp-Leu (SEQ ID NO: 15)

Biotinylated pY peptide ligand containing an aminocaproic acid (Aca) linker used for hcp SH2

Thr-Pro-Pro-His-Leu-Lys-pTyr-Phe-Tyr-Phe-Val-Val-Ser-Asp-Ser-Gly (SEQ ID NO: 16)

Biotinylated pY peptide ligand containing an aminocaproic acid (Aca) linker used for Grb2 SH2

Leu-Pro-Val-Pro-Glu-pTyr-Ile-Asn-Gln-Ser-Val (SEQ ID NO: 26)

Results of Binding Assays:

Tables I and II illustrate the cross reactivity of SH2 antagonists at the indicated SH2 domains. Only Compound 7 has a binding affinity/inhibitory concentration which is greater than fifty-fold higher at the src SH2 domain than the binding affinity/inhibitory concentration at other SH2 domains.

Table I

CROSS REACTIVITY OF Src SH2 DOMAIN ANTAGONISTS AT CLONED HUMAN SH2 DOMAINS ($IC_{50}$)

| Compound | Src | Lck | Fyn | SH-PTP2 | p85 | Grb2 | Hcp |
|---|---|---|---|---|---|---|---|
| 1 | 6 uM | 6 uM | NI | NI | NI | NI | X |
| 2 | NI | NI | NI | NI | NI | NI | 0.9 uM |
| 3 | 16.1 uM | 22.9 uM | NI | NI | NI | NI | 1.2 uM |
| 4 | 40 uM | 163 uM | NI | X | NI | NI | 30 uM |
| 5 | 63 uM | 100 uM | NI | NI | NI | NI | 0.1 uM |
| 6 | 20 uM | NI | NI | X | NI | NI | X |
| 7 | 7.4 uM | 383 uM | NI | NI | NI | NI | NI |
| 8 | 16 uM | 126 uM | NI | NI | NI | NI | >100 uM |
| 9 | 131 uM | 200 uM | NI | NI | NI | NI | 12 uM |
| 10 | X | X | NI | NI | NI | NI | 1.2 uM |

NI-No inhibition observed out to 300uM

X-not tested

Table II

| CROSS REACTIVITY OF Src SH2 DOMAIN ANTAGONISTS AT CLONED HUMAN SH2 DOMAINS (Ki) | | | | | | | |
|---|---|---|---|---|---|---|---|
| Compound | Src | Lck | Fyn | SH-PTP2 | p85 | Grb2 | Hcp |
| 1 | 7 uM | X | NI | NI | NI | NI | X |
| 2 | NI | NI | NI | NI | NI | NI | X |
| 3 | 6 uM | 11 uM | NI | NI | NI | NI | X |
| 4 | 40 uM | 163 uM | NI | XX | NI | NI | X |
| 5 | 28 uM | 149 uM | NI | NI | NI | NI | X |
| 6 | 13 uM | 50 uM | NI | NI | NI | NI | X |
| 7 | 20 uM | 320 uM | NI | NI | NI | NI | X |
| 8 | 12 uM | 360 uM | NI | NI | NI | 330 | X |
| 9 | 55 uM | 146 uM | NI | NI | NI | NI | X |
| 10 | XX | XX | NI | NI | NI | NI | X |
| NI-No inhibition observed below 1000 uM<br>X-not calculated<br>XX-not tested | | | | | | | |

Example 12 Activity of src SH2 Domain Antagonists

4-[4-(4-Methylbenzoyl)benzoyl]phenylacetaldehyde (Compound 4) was tested for its potency to inhibit osteoclast mediated bone resorption in the fetal rat long bone (FRLB) assay as described in Raisz LG (1965) *J Clin Invest* 44:

103-116, Stern PH et al., (1979) *Skeletal Research: An experimental Approach.* New York: Academic Press, 21-59 and Votta BJ et al., (1994) *Bone* 15:533-538).

To perform the experiment timed-pregnant Sprague Dawley rats (Taconic Farms, Germantown, NY) were injected subcutaneously with 200 µCi of $^{45}CaCl_2$ on day 18 of gestation, housed overnight, then anesthetized with Innovar-Vet (Pittman-Moore, Mundelein, IL) and sacrificed by cervical dislocation. Fetuses were removed aseptically and radii and ulnae were dissected free of surrounding soft tissue and cartilaginous ends. The bones were cultured 18-24 hours in $BGJ_b$ medium (Sigma) containing 1 mg/ml bovine serum albumin, then transferred to fresh medium and cultured for an additional 48 hours in the absence or presence of Compound 4. $^{45}$Calcium released into the medium and total calcium in the bones were measured by liquid scintillation spectrometry. Data were expressed as the % $^{45}$calcium released from treated bones as compared to corresponding control bones. Statistical differences were assessed by employing a one-way analysis of variance for non-paired samples. Data are presented as mean ± s.e.m., n=4. The experiment was repeated two times.

4-[4-(4-Methylbenzoyl)benzoyl]phenylacetaldehyde (Compound 4) demonstrated an $IC_{50}$ of 19uM in the above assay.

The above data demonstrates the therapeutic effect of src SH2 domain antagonists in treating a bone resorption disease.

While the preferred embodiments of the invention are illustrated by the above, it is to be understood that the invention is not limited to the precise instructions herein disclosed and that the right to all modifications coming within the scope of the following claims is reserved.

## Claims

1. Use of a compound which:

   a. binds to a human src SH2 domain with a binding affinity which is greater than fifty-fold higher than the binding affinity with which the compound binds to a human lck SH2 domain and a human fyn SH2 domain;
   b. binds to a human hcp SH2 domain with a binding affinity which is greater than fifty-fold lower than the binding affinity with which the compound binds to such src SH2 domain;
   c. binds to a human SH-PTP2 SH2 domain with a binding affinity which is greater than fifty-fold lower than the binding affinity with which the compound binds to such src SH2 domain;
   d. binds to a human p85 SH2 domain with a binding affinity which is greater than fifty-fold lower than the binding affinity with which the compound binds to such src SH2 domain; and
   e. binds to a human Grb2 SH2 domain with a binding affinity which is greater than fifty-fold lower than the binding affinity with which the compound binds to such src SH2 domain.; in the manufacture of a medicament for use in treating a bone resorption disease.

2. A use according to claim 1 wherein the compound binds to a human src SH2 domain with a binding affinity which is greater than fifty-fold higher than the binding affinity with which the compound binds to a human lck SH2 domain and a human fyn SH2 domain.

3. A use according to claim 1 wherein the compound:

   a. binds to a human src SH2 domain with a binding affinity which is greater than one hundred-fold higher than the binding affinity with which the compound binds to a human lck SH2 domain and a human fyn SH2 domain;
   b. binds to a human hcp SH2 domain with a binding affinity which is greater than one hundred-fold lower than the binding affinity with which the compound binds to such src SH2 domain;
   c. binds to a human SH-PTP2 SH2 domain with a binding affinity which is greater than one hundred-fold lower than the binding affinity with which the compound binds to such src SH2 domain;
   d. binds to a human p85 SH2 domain with a binding affinity which is greater than one hundred-fold lower than the binding affinity with which the compound binds to such src SH2 domain; and
   e. binds to a human Grb2 SH2 domain with a binding affinity which is greater than one hundred-fold lower than the binding affinity with which the compound binds to such src SH2 domain.

4. A use according to claim 3 wherein the compound binds to a human src SH2 domain with a binding affinity which is greater than one hundred-fold higher than the binding affinity with which the compound binds to a human lck SH2 domain and a human fyn SH2 domain.

5. Use of a compound which:

   a. binds to a human src SH2 domain with a binding affinity which is greater than fifty-fold higher than the binding affinity with which the compound binds to a human lck SH2 domain and a human fyn SH2 domain;
   b. binds to a human hcp SH2 domain with a binding affinity which is greater than fifty-fold lower than the binding affinity with which the compound binds to such src SH2 domain;
   c. binds to a human SH-PTP2 SH2 domain with a binding affinity which is greater than fifty-fold lower than the binding affinity with which the compound binds to such src SH2 domain;
   d. binds to a human p85 SH2 domain with a binding affinity which is greater than fifty-fold lower than the binding affinity with which the compound binds to such src SH2 domain; and
   e. binds to a human Grb2 SH2 domain with a binding affinity which is greater than fifty-fold lower than the binding affinity with which the compound binds to such src SH2 domain; in the manufacture of a medicament for use in treating osteoporosis.

6. A use according to claim 5 wherein the compound binds to a human src SH2 domain with a binding affinity which is greater than fifty-fold higher than the binding affinity with which the compound binds to a human lck SH2 domain and a human fyn SH2 domain.

7. A use according to claim 5 wherein the compound:

   a. binds to a human src SH2 domain with a binding affinity which is greater than one hundred-fold higher than

the binding affinity with which the compound binds to a human lck SH2 domain and a human fyn SH2 domain;
b. binds to a human hcp SH2 domain with a binding affinity which is greater than one hundred-fold lower than the binding affinity with which the compound binds to such src SH2 domain;
c. binds to a human SH-PTP2 SH2 domain with a binding affinity which is greater than one hundred-fold lower than the binding affinity with which the compound binds to such src SH2 domain;
d. binds to a human p85 SH2 domain with a binding affinity which is greater than one hundred-fold lower than the binding affinity with which the compound binds to such src SH2 domain; and
e. binds to a human Grb2 SH2 domain with a binding affinity which is greater than one hundred-fold lower than the binding affinity with which the compound binds to such src SH2 domain.

**8.** A use according to claim 7 wherein the compound binds to a human src SH2 domain with a binding affinity greater than one hundred-fold higher than the binding affinity with which the compound binds to a human lck SH2 domain and a human fyn SH2 domain.

**9.** Use of a compound which:

a. binds to a human src SH2 domain with a binding affinity which is greater than fifty-fold higher than the binding affinity with which the compound binds to a human lck SH2 domain and a human fyn SH2 domain;
b. binds to a human hcp SH2 domain with a binding affinity which is greater than fifty-fold lower than the binding affinity with which the compound binds to such src SH2 domain;
c. binds to a human SH-PTP2 SH2 domain with a binding affinity which is greater than fifty-fold lower than the binding affinity with which the compound binds to such src SH2 domain;
d. binds to a human p85 SH2 domain with a binding affinity which is greater than fifty-fold lower than the binding affinity with which the compound binds to such src SH2 domain; and
e. binds to a human Grb2 SH2 domain with a binding affinity which is greater than fifty-fold lower than the binding affinity with which the compound binds to such src SH2 domain; in the manufacture of a medicament for use in impairing the function of osteoclast.

**10.** A use according to claim 9 wherein the compound binds to a human src SH2 domain with a binding affinity which is greater than fifty-fold higher than the binding affinity with which the compound binds to a human lck SH2 domain and a human fyn SH2 domain.

**11.** A use according to claim 9 wherein the compound:

a. binds to a human src SH2 domain with a binding affinity which is greater than one hundred-fold higher than the binding affinity with which the compound binds to a human lck SH2 domain and a human fyn SH2 domain;
b. binds to a human hcp SH2 domain with a binding affinity which is greater than one hundred-fold lower than the binding affinity with which the compound binds to such src SH2 domain;
c. binds to a human SH-PTP2 SH2 domain with a binding affinity which is greater than one hundred-fold lower than the binding affinity with which the compound binds to such src SH2 domain;
d. binds to a human p85 SH2 domain with a binding affinity which is greater than one hundred-fold lower than the binding affinity with which the compound binds to such src SH2 domain; and
e. binds to a human Grb2 SH2 domain with a binding affinity which is greater than one hundred-fold lower than the binding affinity with which the compound binds to such src SH2 domain.

**12.** A use according to claim 11 wherein the compound binds to a human src SH2 domain with a binding affinity which is greater than one hundred-fold higher than the binding affinity with which the compound binds to a human lck SH2 domain and a human fyn SH2 domain.

European Patent Office

# EUROPEAN SEARCH REPORT

Application Number
EP 96 20 0270

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | BONE, vol. 8, no. 2, 1987, pages 65-70, XP000571953 "Exogeneous Triiodothyronine activates bone remodelling" * the whole document * --- | 1-12 | A61K31/50 A61K31/435 A61K31/12 A61K31/195 A61K31/165 A61K31/40 |
| A | J.BONE MINER.RES., vol. 8, no. 12, 1993, pages 1475-81, XP002004195 "Thyroid hormones increase insulin-like growth factor I content in the medium of rat bone tissue" * the whole document * ----- | 1-12 | |
| | | | TECHNICAL FIELDS SEARCHED (Int.Cl.6) |
| | | | A61K |

The present search report has been drawn up for all claims

| Place of search | Date of completion of the search | Examiner |
|---|---|---|
| THE HAGUE | 29 May 1996 | Gerli, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)